# EUROPEAN PATENT SPECIFICATION

(11) **EP 4 363 433 B1**
(45) Date of publication and mention of the grant of the patent: **08.10.2025**
(21) Application number: 22746974.9
(22) Date of filing: 04.07.2022
(51) Int. Cl.: C07K 1/00, C07K 5/062, C07K 5/078, C07K 5/09, C07D 209/16

(54) **PEPTIDE SYNTHESIS METHOD INVOLVING STERICALLY HINDERED MIXED ANHYDRIDE INTERMEDIATE**
PEPTIDSYNTHESEVERFAHREN MIT EINEM STERISCH GEHINDERTEN MISCHANHYDRIDZWISCHENPRODUKT
PROCÉDÉ DE SYNTHÈSE DE PEPTIDES IMPLIQUANT UN INTERMÉDIAIRE ANHYDRIDE MIXTE STÉRIQUEMENT ENCOMBRÉ

(30) Priority: 02.07.2021 GB 202109626
(43) Date of publication of application: 08.05.2024
(73) Proprietor: Amicoat AS, 9019 Tromsø (NO)
(72) Inventor: EKSTEEN, Jacobus Johannes, 9014 Tromsø (NO); SVENDSEN, John Sigurd, 9019 Tromsø (NO); BORGHESE, Sophie, 1120 Brussels (BE); MALMEDY, Florence, 1120 Brussels (BE); BOUSMANNE, Martin, 1200 Woluwe Saint-Lambert (BE)
(74) Representative: Dehns
(86) International application number: PCT/EP2022/068484
(87) International publication number: WO 2023/275410

(56) References cited:
- WO-A1-2010/038040

## Description

### Technical Field

The invention is directed to a method of making a target peptide.

### Background

At laboratory scale, many coupling strategies are available for the production of peptides. However, most of these are too expensive for commercial scale production. Coupling reactions between amino acids are, almost exclusively, facilitated by activation of the carboxylic acid of the incoming amino acid. For example, *O*-(1H-Benzotriazol-1-yl)-N,N,N',N'-tetramethyluronium hexafluorophosphate (HTBU) is an efficient activator that gives minimal racemization. Synthesis of peptides comprising tri-tert-butyl-tryptophan by HBTU-mediated coupling is for example disclosed in WO2010/038040 A1.
However, the cost of HTBU is high.

It would be desirable to be able to use lower cost activators without compromising on selectivity or reaction rate. However, lower cost activators are not suitable for all peptide coupling strategies. In particular, depending on the candidate amino acid to be activated, some activators may not allow coupling at acceptable rates or with acceptable selectivity. Slow reactions in particular may be associated with undesirable racemization/epimerisation.

As illustrated in Figure 22 of Rehman et al., Side reactions in peptide synthesis: An overview, International Journal of Pharmaceutical Research & Technology, Volume - 10, 2018, when a candidate amino acid contains a sterically bulky side chain, activators that form mixed anhydride intermediates do not usually provide acceptable selectivity, since the amino nucleophile has a better chance to attack on the undesired carbonyl group which does not result in amide bond formation.

### Summary of the invention

The present inventors have surprisingly found that mixed anhydride peptide synthesis can be used and will provide yields suitable for a commercial process even when the peptides incorporate an amino acid with a sterically bulky side chain. Thus, in one aspect, the invention provides a method for making a target peptide comprising reacting a mixed anhydride compound of Formula (I) with a second moiety which is an amino acid or peptide;
wherein Formula (I) has the structure: 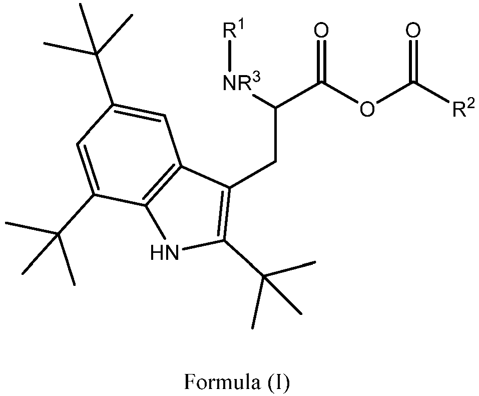
wherein R¹ is a protecting group, a peptide or an amino acid;
wherein R² is tert-butyl, isobutoxy, tert-butoxy, isobutyl, isopropoxy, isopropyl, or ethoxy; and
wherein R³ is H or an alkylsilyl group.

In another aspect, the invention provides a method of peptide synthesis comprising reacting a mixed anhydride compound of Formula (I) with a second moiety which is an amino acid or peptide. The method may be a step in the synthesis of a target peptide. Embodiments of other aspects of the invention described herein apply, *mutatis mutandis,* to this aspect of the invention.

In another aspect, the invention is directed to a compound of Formula (I). Embodiments of other aspects of the invention described herein apply, *mutatis mutandis,* to this aspect of the invention.

### Brief Description of the Figures

Figure 1 shows two possible reactions between an exemplary mixed anhydride compound and an amino group of an exemplary second moiety. The desired reaction (upper arrow) involves the carbonyl closest to the tri-*tert*-butyl-tryptophan (Tbt) side chain. The undesired reaction (bottom arrow) involves reaction at the "wrong" carbonyl group of the mixed anhydride compound and does not result in the formation of the desired amide bond. The second moiety in Figure 1 comprises an arginine residue and R² in Figure 1 represents the rest of the second moiety.
Figures 2A-4A show UPLC-PDA traces of the activation and coupling of Fmoc-Aib-OH (Figure 2A), Fmoc-Tle-OH (Figure 3A), and Fmoc-Ile-OH (Figure 4A) to H-Arg-OMe using pivaloyl chloride.
Figures 2B-4B show UPLC-MS spectra after completion of the coupling reaction of Fmoc-Aib-OH (Figure 2B), Fmoc-Tle-OH (Figure 3B), and Fmoc-Ile-OH (Figure 4B) to H-Arg-OMe using pivaloyl chloride.
Figure 5 shows UPLC-PDA traces (Figure 5A) of the activation and coupling of Fmoc-Tbt-OH to H-Arg-OMe using pivaloyl chloride and UPLC-MS spectra (Figure 5B) after completion of the coupling reaction.
Figure 6 shows UPLC-PDA traces (Figure 6A) of the activation and coupling of Fmoc-Tbt-OH to H-Arg-OMe using isobutylchloroformate and UPLC-MS spectra (Figure 6B) after completion of the coupling reaction.

### Detailed Description

The method of the invention is for making a target peptide and involves reacting a mixed anhydride compound of Formula (I) with a second moiety which is an amino acid or a peptide.

The reaction results in the formation of an amide bond between the carboxyl group of the Tbt residue in Formula (I) and the amino group of the second moiety. The reaction between the mixed anhydride compound of Formula (I) and the second moiety may form the target peptide or a precursor to the target peptide. In some cases, subsequent removal of protecting groups or further peptide coupling reactions may be required to convert the precursor into the target peptide.

As set out in the Summary of the Invention, one aspect of the invention is directed to a compound of Formula (I).

### Mixed anhydride compound of Formula (I)

Formula (I) comprises an extremely sterically bulky tri-*tert*-butyl-tryptophan (Tbt) residue and has the structure:

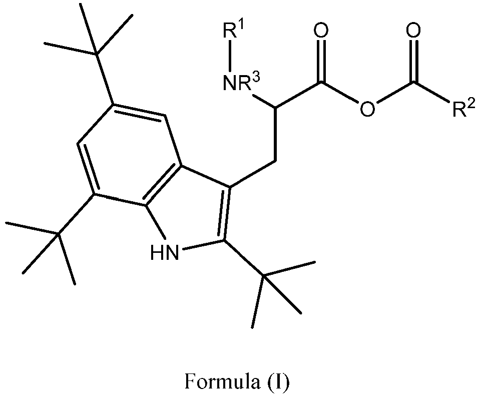

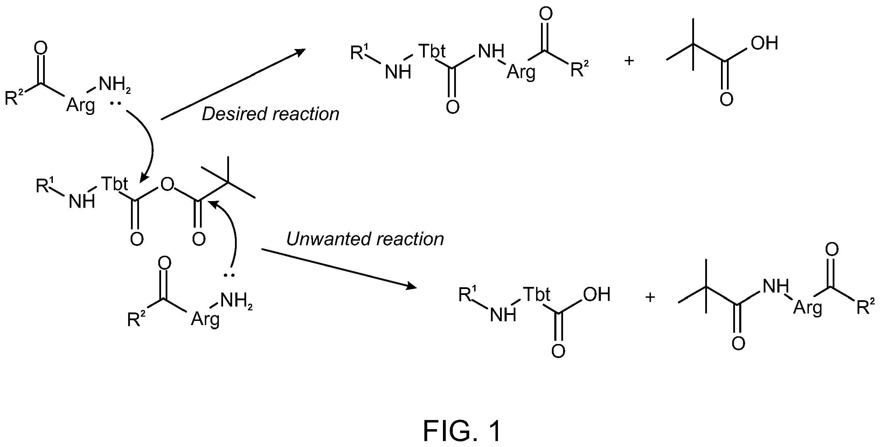

As illustrated in Figure 1, there are two carbonyl groups in Formula (I) that an amino nucleophile in the second moiety could attack. Attack at the "correct" carbonyl (the Tbt carbonyl) results in the desired formation of the amide bond whereas attack at the "wrong" carbonyl (the carbonyl adjacent to R² which may be derived from the activator of Formula (III) as set out below) does not result in the formation of the amide bond.

The inventors have unexpectedly found that, in spite of the very high steric bulk of the Tbt side chain, the mixed anhydride of Formula (I) reacts with amino acids or peptides quickly, thereby reducing undesirable epimerization, and with good selectivity for the "correct" carbonyl (the Tbt carbonyl). In comparative experiments employing amino acids that are far less sterically hindered than Tbt, the coupling reactions were slow and significant amounts of product resulting from reaction at the "wrong" carbonyl (the carbonyl adjacent to R²) were observed.

In Formula (I), R¹ is: a protecting group, a peptide or an amino acid. Optionally, the peptide or the amino acid may themselves comprise one or more protecting groups, such as on their *N*-terminal amino groups.

As used herein the term peptide includes peptomimetics, although true peptides are preferred. A peptidomimetic is typically characterised by retaining the polarity, three dimensional size and functionality (bioactivity) of its peptide equivalent but wherein the peptide bonds have been replaced, often by more stable linkages. By 'stable' is meant more resistant to enzymatic degradation by hydrolytic enzymes. Generally, the bond which replaces the amide bond (amide bond surrogate) conserves many of the properties of the amide bond, e.g. conformation, steric bulk, electrostatic character, possibility for hydrogen bonding etc. Chapter 14 of "Drug Design and Development", Krogsgaard, Larsen, Liljefors and Madsen (Eds) 1996, Horwood Acad. Pub provides a general discussion of techniques for the design and synthesis of peptidomimetics. In the present case, where the target peptide reacts with a membrane rather than the specific active site of an enzyme, some of the problems described of exactly mimicing affinity and efficacy or substrate function are not relevant and a peptidomimetic can be readily prepared based on a given peptide structure or a motif of required functional groups. Suitable amide bond surrogates include the following groups: N-alkylation (Schmidt, R. et al., Int. J. Peptide Protein Res., 1995, 46,47), retro-inverse amide (Chorev, M and Goodman, M., Acc. Chem. Res, 1993, 26, 266), thioamide (Sherman D.B. and Spatola, A.F. J. Am. Chem. Soc., 1990, 112, 433), thioester, phosphonate, ketomethylene (Hoffman, R.V. and Kim, H.O. J. Org. Chem., 1995, 60, 5107), hydroxymethylene, fluorovinyl (Allmendinger, T. et al., Tetrahydron Lett., 1990, 31, 7297), vinyl, methyleneamino (Sasaki, Y and Abe, J. Chem. Pharm. Bull. 1997 45, 13), methylenethio (Spatola, A.F., Methods Neurosci, 1993, 13, 19), alkane (Lavielle, S. et. al., Int. J. Peptide Protein Res., 1993, 42, 270) and sulfonamido (Luisi, G. et al. Tetrahedron Lett. 1993, 34, 2391).

The term 'amino acid' may thus conveniently be used herein to refer to the equivalent sub-units of a peptidomimetic compound. Moreover, peptidomimetics may have groups equivalent to the R groups of amino acids.

As is discussed in the text book referenced above, as well as replacement of amide bonds, peptidomimetics may involve the replacement of larger structural moieties with di- or tripeptidomimetic structures and in this case, mimetic moieties involving the peptide bond, such as azole-derived mimetics may be used as dipeptide replacements. Peptidomimetics and thus peptidomimetic backbones wherein the amide bonds have been replaced as discussed above are, however, preferred.

Suitable peptidomimetics include reduced peptides where the amide bond has been reduced to a methylene amine by treatment with a reducing agent e.g. borane or a hydride reagent such as lithium aluminium-hydride. Such a reduction has the added advantage of increasing the overall cationicity of the molecule.

Other peptidomimetics include peptoids formed, for example, by the stepwise synthesis of amide-functionalised polyglycines. Some peptidomimetic backbones will be readily available from their peptide precursors, such as peptides which have been permethylated, suitable methods are described by Ostresh, J.M. et al. in Proc. Natl. Acad. Sci. USA(1994) 91, 11138-11142. Strongly basic conditions will favour N-methylation over O-methylation and result in methylation of some or all of the nitrogen atoms in the peptide bonds and the N-terminal nitrogen. Preferred peptidomimetic backbones include polyesters, polyamines and derivatives thereof as well as substituted alkanes and alkenes. The peptidomimetics will preferably have N and C terminii which may be modified as discussed herein.

Preferably, the term "amino acid" as used herein refers to proteinogenic (genetically encoded) amino acids. Preferably, the term peptide in R¹ and the second moiety refers to a peptide formed from proteinogenic amino acids.

R¹ typically comprises 1 to 10 amino acids, preferably 1 to 5 or 1 to 3 amino acids, most preferably 1 amino acid.

A wide choice of protecting groups suitable for amino acids are known (see, for example, Greene, T. W. and Wuts, P. G. M., Protective Groups in Organic Synthesis, 3rd ed., Wiley: New York, 1999 and Isidro-Llobet et al., Chem. Rev. 2009, 109, 6, 2455-2504).

Suitable amine protecting groups include carbobenzoxy (also known as benzyloxycarbonyl and designated Z or Cbz), t-butoxycarbonyl (also designated Boc), 4-methoxy-2,3,6-trimethylbenzene sulphonyl (Mtr), 9-fluorenylmethoxy-carbonyl (also designated Fmoc) and 2,2,2-trichloroethoxycarbonyl (Troc). These protecting groups may themselves be R¹ or one or more of these protecting groups may be present on R¹ when R¹ is a peptide or an amino acid.

Suitable carboxyl protecting groups which may, for example be employed include readily cleaved ester groups such as benzyl (Bn), p-nitrobenzyl (pNb), pentachlorophenyl (PCIP), pentafluorophenyl (Pfp) or t-butyl (tBu) groups as well as the coupling groups on solid supports, for example methyl groups linked to polystyrene. Other suitable carboxyl protecting groups include 4-{*N*-[1-(4,4-dimethyl-2,6-dioxocyclohexylidene)-3-methylbutyl]amino}benzyl ester (Dmab), allyloxycarbonyl (Alloc) and 2-phenylisopropyl (2-PhiPr).

Thiol protecting groups include p-methoxybenzyl (Mob), trityl (Trt), acetamidomethyl (Acm), *tert-*butyl (tBu), *tert*-butylthio (tButhio) and monomethoxytrityl (Mmt) groups.

Amine protecting groups such as Boc and carboxyl protecting groups such as tBu may be removed simultaneously by acid treatment, for example with trifluoroacetic acid. Thiol protecting groups such as Trt may be removed selectively using an oxidation agent such as iodine.

Preferably, R¹ is a peptide or an amino acid, optionally comprising one or more protecting groups, such as on its *N*-terminal amino group. The amino acid may be a cationic amino acid AA₁.

Preferably, R¹ is a cationic amino acid AA₁ optionally comprising one or more protecting groups, such as on its N-terminal amino group.

AA₁ is preferably, lysine or arginine but may be histidine or any non genetically coded or modified amino acid carrying a positive charge at pH 7.0. Suitable non-genetically coded amino acids and modified amino acids which can provide a cationic amino acid include analogues of lysine, arginine and histidine such as homolysine, ornithine, diaminobutyric acid, diaminopimelic acid, diaminopropionic acid and homoarginine as well as trimethylysine and trimethylornithine, 4-aminopiperidine-4-carboxylic acid, 4-amino-1-carbamimidoylpiperidine-4-carboxylic acid and 4-guanidinophenylalanine.

Most preferably, R¹ is arginine optionally comprising one or more protecting groups, such as on its N-terminal amino group.

In Formula (I), R² is selected from *tert-*butyl*,* isobutoxy, *tert-*butoxy*,* isobutyl, isopropoxy, isopropyl, or ethoxy. Preferably, R² is selected from *tert-*butyl*,* isobutoxy, *tert-*butoxy*,* isobutyl, isopropoxy, or isopropyl. More preferably, R² is *tert-*butyl or isobutoxy, most preferably R² is *tert-*butyl*.*

In other preferred cases, R² is one of the alkyl groups defined above, i.e. *tert-*butyl, isobutyl or isopropyl, preferably *tert-*butyl*.*

In Formula (I), R³ is selected from H or an alkylsilyl group, such as a mono(C₁-C₆ alkyl)silyl, di(C₁-C₆ alkyl)silyl or tri(C₁-C₆ alkyl)silyl group. Preferably, the alkylsilyl group is a tri(C₁-C₆ alkyl)silyl group, more preferably a tri(C₁-C₃ alkyl)silyl group. Each alkyl group may be the same or different, preferably the same. If present as R³, the alkylsilyl group is preferably trimethyl silyl.

R³ is preferably H. That is, the compound of Formula (I) preferably has the structure

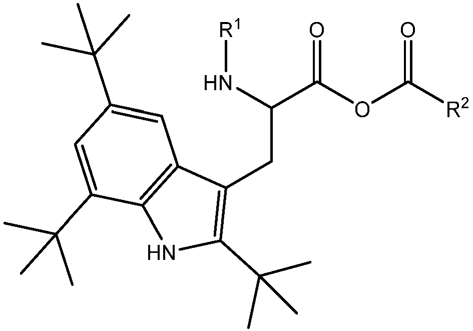

### Second moiety

The second moiety is an amino acid or a peptide. The amino acid or peptide of the second moiety may optionally contain one or more protecting groups and/or a C-terminal capping group. The amino acid or peptide of the second moiety may optionally be silylated. Suitable silylating agents are disclosed in WO 2009/065836. For example, suitable silylating agents are N-trialkylsilyl amines or N-trialkylsilyl amides, such as those selected from the group consisting of: N,O-bis(trimethylsilyl)acetamide, N,O-bis(trimethylsilyl)trifluoroacetamide, hexamethyldisilazane, N-methyl-N-trimethylsilylacetamide (MSA), N-methyl-N-trimethylsilyltrifluoroacetamide, N-(trimethylsilyl)acetamide, N-(trimethylsilyl)diethylamine, N-(trimethylsilyl)dimethylamine, 1-(trimethylsilyl)imidazole and 3-(trimethylsilyl)-2-oxazolidone. Silylation may improve solubility of the second moiety, for example in polar organic solvents such as polar aprotic solvents, e.g. dimethylacetamide. During silylation one or more functional groups in the second moiety having an active hydrogen, such as amino, hydroxyl, mercapto or carboxyl groups, react with the silylating agent. The silylated second moiety then comprises one or more silyl groups (such as trialkylsilyl, typically tri(C₁-C₃)alkyl groups such as trimethylsilyl) bonded to said functional groups. The amino acid or peptide of the second moiety may optionally be provided in the form of a salt, such as a hydrochloride, trifluoroacetate or acetate salt.

The second moiety typically comprises a reactive amino group, in particular an alpha-amino group. The alpha-amino group is preferably not protonated so that it is available to act as a nucleophile.

The second moiety typically comprises 1 to 10 amino acids, preferably 1 to 5 or 1 to 3 amino acids, most preferably 1 amino acid.

As set out above, suitable protecting groups for amino acids are well known and the protecting groups listed above may also be used in the second moiety.

Suitable C-terminal capping groups are of formula -X-Y-Z, wherein the left hyphen denotes the point of attachment to the carbon of the C-terminal carbonyl and X, Y and Z are defined as for Formula (IV) below. In other words, if present, capping group -X-Y-Z is attached to the remainder of the second moiety as follows: 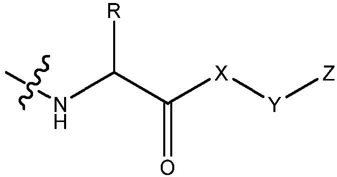 wherein R denotes the side chain of the C-terminal amino acid. Preferably, -X-Y-Z together is the group -NHCH₂CH₂Ph.

Preferably, the second moiety is a compound of Formula (IV):

AA₃-X-Y-Z (IV)

wherein AA₃ is a cationic amino acid, preferably lysine or arginine but may be histidine or any non genetically coded or modified amino acid carrying a positive charge at pH 7.0;
X is a N atom, which may be, but preferably is not, substituted by a branched or unbranched C₁-C₁₀ alkyl or aryl group (such as a C₄-C₁₀ aryl group), e.g. methyl, ethyl or phenyl, and this alkyl or aryl group may incorporate up to 2 heteroatoms selected from N, O and S;
Y represents a group selected from -Rₐ-R_{b}-, -Rₐ-R_{b}-R_{b}- and -R_{b}-R_{b}-Rₐ- wherein
Rₐ is C, O, S or N, preferably C, and
R_{b} is C; each of Rₐ and R_{b} may be substituted by C₁-C₄ alkyl groups or unsubstituted, preferably Y is -Rₐ-R_{b}- (in which Rₐ is preferably C) and preferably this group is not substituted, when Y is -Rₐ-R_{b}-R_{c}- or R_{b}-R_{b}-Rₐ- then preferably one or more of Rₐ and R_{b} is substituted; and
Z is a group comprising 1 to 3 cyclic groups each of 5 or 6 non-hydrogen atoms (preferably C atoms), 2 or more of the cyclic groups may be fused; one or more of the rings may be substituted and these substitutions may, but will typically not, include polar groups, suitable substituting groups include halogens, preferably bromine or fluorine and C₁-C₄ alkyl groups; the Z moiety incorporates a maximum of 15 non-hydrogen atoms, preferably 5-12, most preferably it is phenyl;
the bond between Y and Z is a covalent bond between Rₐ or R_{b} of Y and a non-hydrogen atom of one of the cyclic groups of Z.

The compound of Formula (IV) may optionally contain one or more protecting groups and/or be silylated. The discussion of silylation and suitable silylating agents above applies equally when the second moiety is a compound of Formula (IV).

Suitable non-genetically coded amino acids and modified amino acids which can provide a cationic amino acid include analogues of lysine, arginine and histidine such as homolysine, ornithine, diaminobutyric acid, diaminopimelic acid, diaminopropionic acid and homoarginine as well as trimethylysine and trimethylornithine, 4-aminopiperidine-4-carboxylic acid, 4-amino-1-carbamimidoylpiperidine-4-carboxylic acid and 4-guanidinophenylalanine.

Preferably, Y is -Rₐ-R_{b}- as defined above, more preferably wherein Rₐ and R_{b} are unsubstituted, most preferably wherein Rₐ and R_{b} are both carbon atoms. In other words, Y is most preferably -CH₂CH₂-.

Preferably, -X-Y-Z together is the group -NHCH₂CH₂Ph.

Most preferably, AA₃ is arginine.

In other preferred cases, the second moiety is an amino acid comprising AA₃ or a peptide comprising AA₃ as the N-terminal amino acid, optionally wherein the amino acid or peptide comprise one or more protecting groups and/or a C-terminal capping group. The definitions of AA₃ above in the context of Formula (IV) apply equally in this case. The C-terminal capping group may have the structure -X-Y-Z, and the preferred definitions of each of -X-Y-Z set out above also apply equally to this case. The amino acid comprising AA₃ or the peptide comprising AA₃ as the N-terminal amino acid may optionally be silylated, and the discussion of silylation and suitable silylating agents above applies equally.

The compounds of the invention and those used and made in/by the methods of the invention (e.g. of Formula (I), the target peptide and the second moiety) may include all enantiomeric forms, both D and L amino acids and enantiomers resulting from chiral centers within the amino acid R groups and moieties Y or Z.

More preferably, the second moiety is arginine, optionally comprising one or more protecting groups and/or a C-terminal capping group. In this case, the C-terminal capping group may be of formula -X-Y-Z, and is preferably -NHCH₂CH₂Ph.

The reaction between the compound of Formula (I) and the second moiety may be carried out in a suitable solvent. Suitable solvents include aqueous or non-aqueous solvents. Examples of suitable solvents include water; DCM; DMF; methanol; *N,N*-dimethylacetamide (DMA); acetonitrile (ACN); N-methylpyrrolidone and dimethylsulfoxide; 2-methyltetrahydrofuran (MeTHF) or a mixture thereof, such as a mixture of DMF and methanol. Preferably, the solvent is DMF or a mixture of DMF and methanol. Other preferred solvents include ACN, MeTHF, DMA and mixtures thereof.

The second moiety is preferably added to the compound of Formula (I) in the form of a solution. In some preferred cases, the second moiety may be added to the compound of Formula (I) in the form of a solution comprising DMA solvent.

Prior to the reaction with the compound of Formula (I), the second moiety may optionally be treated with an acid to improve solubility, such as in DMA solvent. Any suitable acid may be used. For example, suitable acids include hydrochloric acid, trifluoroacetic acid, acetic acid. The acid may be provided in aqueous or non-aqueous form. Optionally, approximately 3 equivalents of acid per equivalent of the second moiety may be added, such as from 2.5 to 3.5 equivalents or from 2.9 to 3.3 equivalents of the acid per equivalent of second moiety.

Surprisingly the reaction is fast despite the very high steric bulk of the Tbt side chain, which may reduce epimerization and thereby facilitate purification.

The reaction may be carried out at room temperature and pressure, e.g. 20-25 °C and 1 atmosphere of pressure. The reaction may also be carried out at lower temperatures. For example, the reaction may be carried out at any temperature from approximately -20 °C to room temperature, such as from -15 °C to 25 °C, preferably from -10 °C to 20 °C.

The reaction between the compound of Formula (I) and the second moiety may optionally be carried out in the presence of a base. Optionally, one or more bases may be used. The use of a base may ensure that the α-amino group is not protonated. Thus, the base(s) may be added in an amount sufficient to ensure that the α-amino group is not protonated so that it is available to act as a nucleophile. For example, one molar equivalent of base per molar equivalent of second moiety may be used. Where the second moiety has previously been treated with an acid as set out above, more than one equivalent of base may be required. For example, when the second moiety comprises an amino acid having a basic side group and has been treated with approximately 3 equivalents of acid per equivalent of second moiety (such as from 2.5 to 3.5 equivalents or from 2.9 to 3.3 equivalents of acid per equivalent of second moiety), approximately 2 equivalents of acid (such as from 1.5-2.5 or 1.8-2.2 equivalents of base) per equivalent of second moiety may be used. Suitable bases include: DIPEA, N-methylmorpholine, pyridine, trimethylamine and 2,4,6-trimethylpyridine. Preferably, the base is DIPEA. If used, the base may preferably be added after mixing the second moiety and the compound of Formula (I).

The method of the invention may further comprise one or more purification steps, such as to remove side products resulting from the reaction of the second moiety at the "wrong" carbonyl of Formula (I).

### Tbt-activation and activator

The mixed anhydride compound of Formula (I) may be prepared by reacting a first moiety of Formula (II) with an activator of Formula (III) in the presence of a base. Thus, in one aspect, the method of the invention comprises first preparing the compound of Formula (I) by reacting a first moiety of Formula (II) with an activator of Formula (III) in the presence of a base.

The compound of Formula (I) may optionally be isolated before reaction with the second moiety. Preferably, the compound of Formula (I) is not isolated before reaction with the second moiety.

Formula (II) has the structure: 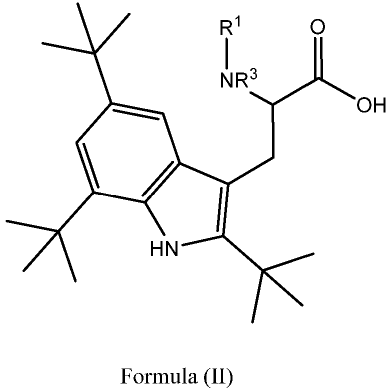 wherein R¹ is a protecting group, a peptide or an amino acid. The discussion of R¹ and R³ above in connection with Formula (I) applies equally to Formula (II). For example, R³ is preferably H and so Formula (II) preferably has the structure

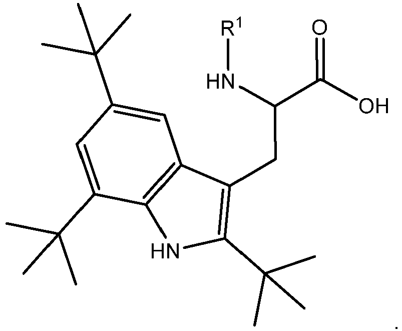

Formula (III) has the structure: 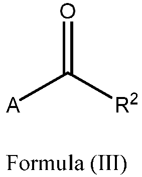 wherein R² is *tert*-butyl, isobutoxy, *tert*-butoxy, isobutyl, isopropoxy, isopropyl, or ethoxy. Preferably, R² is selected from tert-butyl, isobutoxy, tert-butoxy, isobutyl, isopropoxy, or isopropyl. More preferably, R² is *tert*-butyl or isobutoxy, most preferably R² is *tert*-butyl.

In other preferred cases, R² is one of the alkyl groups defined above, i.e. *tert-*butyl, isobutyl or isopropyl, preferably *tert*-butyl.

A is a halogen, such as Cl, Br or I. Preferably, A is Cl.

The activator of Formula (III) is preferably pivaloyl chloride or isobutylchloroformate, more preferably, pivaloyl chloride.

Any suitable base may be used, for example suitable bases include N,N-diisopropylethylamine (DIPEA), *N*-methylmorpholine, pyridine, and triethylamine. Preferably, the base is *N,N*-diisopropylethylamine (DIPEA).

The reaction between the compounds of Formula (II) and Formula (III) may be carried out in a non-aqueous solvent. Suitable solvents include DCM; DMF; N,N-dimethylacetamide; acetonitrile (ACN); N-methylpyrrolidone and dimethylsulfoxide; MeTHF; or a mixture thereof. Preferably, the solvent is DMF. In other preferred cases, the solvent is a mixture of ACN and MeTHF.

The reaction between the compounds of Formula (II) and Formula (III) may be carried out at room temperature and pressure, e.g. 20-25 °C and 1 atmosphere of pressure. The reaction may also be carried out at lower temperatures. For example, the reaction may be carried out at any temperature from approximately -20 °C to room temperature, such as from -15 °C to 25 °C, preferably from -10 °C to 20 °C.

The method of the invention may further comprise preparing the compound of Formula (II). For example, when R¹ is a peptide or an amino acid, the method may further comprise coupling said peptide or amino acid to the Tbt residue. Any suitable peptide coupling technique may be used to link the R¹ group to the Tbt residue. In some cases, the compound of Formula (II) may be prepared by pre-activating the R¹ amino acid or peptide, such as with pivaloyl chloride or isobutylchloroformate, and then coupling with Tbt, optionally silylated Tbt. Peptide production methods involving silylated peptides are disclosed in WO2009/065836.

The method of the invention may also further comprise preparing the second moiety. For example, when the second moiety comprises the C-terminal capping group, such as a capping group having formula -X-Y-Z as defined above (e.g. -NHCH₂CH₂Ph), the process may comprise activating the C-terminal carboxylic acid group of the second moiety, which typically comprises an amino protecting group such as Cbz, and then coupling to H-X-Y-Z (e.g. H₂NCH₂CH₂Ph). Suitable activators for this step include pivaloyl chloride or isobutylchloroformate.

### Target peptide

A target peptide according to the present invention will typically have a chain length of up to 20 amino acids. Preferably, target peptides are 2 to 10, 3 to 7 or 3 to 5, e.g. 3 amino acids in length.

The target peptide is preferably an antimicrobial peptide.

Preferably, the target peptide is a compound of Formula (V)

AA₁-AA₂-AA₃-X-Y-Z (V)

wherein:
each AA₁ and AA₃ is independently a cationic amino acid, preferably lysine or arginine but may be histidine or any non-genetically coded or modified amino acid carrying a positive charge at pH 7.0;
AA₂ is Tbt, i.e. 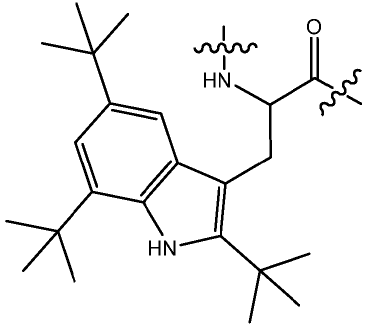 wherein the left hand wiggly bond denotes the point of attachment to AA₁ and the right hand wiggly bond denotes the point of attachment to AA₃-X-Y-Z; and
X, Y and Z are as defined above.

Non-genetically coded or modified amino acids that are suitable as AA₁ and/or AA₃ are set out above.

The compounds of Formula (V) are antimicrobial peptides and are disclosed in WO2009/081152.

The target peptide may include all enantiomeric forms, both D and L amino acids and enantiomers resulting from chiral centers within the amino acid R groups and moieties Y or Z, when present.

Preferably, the target peptide is Arg-Tbt-Arg-NHCH₂CH₂Ph, i.e. the compound 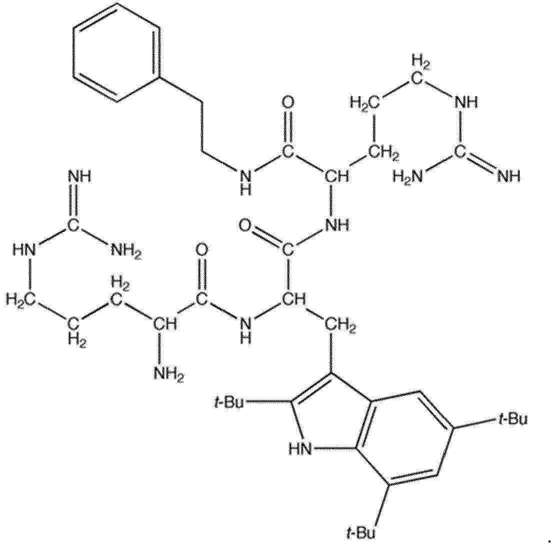 Most preferably, the target peptide has the following structure: 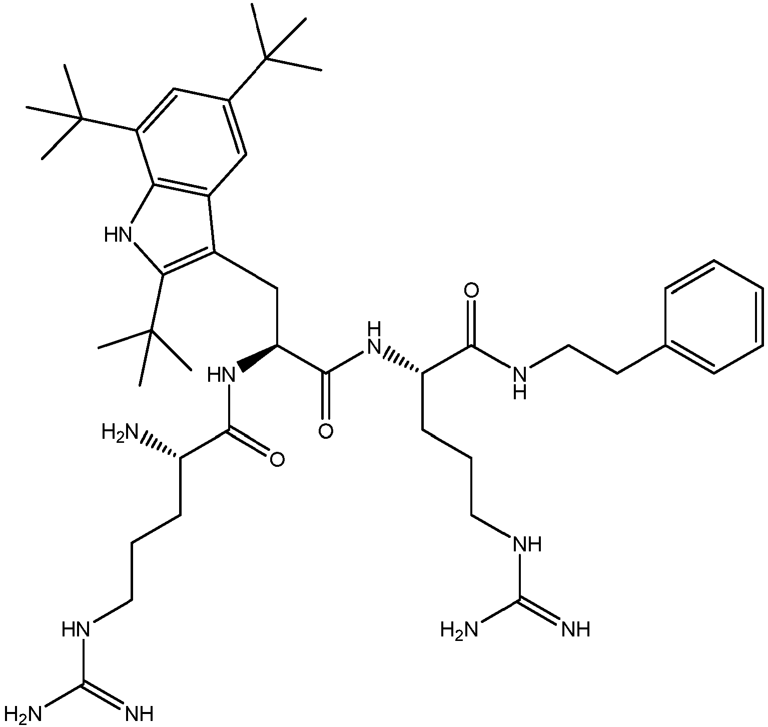 which is also referred to herein as AMC-109.

Scheme 1 shows an exemplary synthesis strategy for the production of AMC-109 according to the invention.

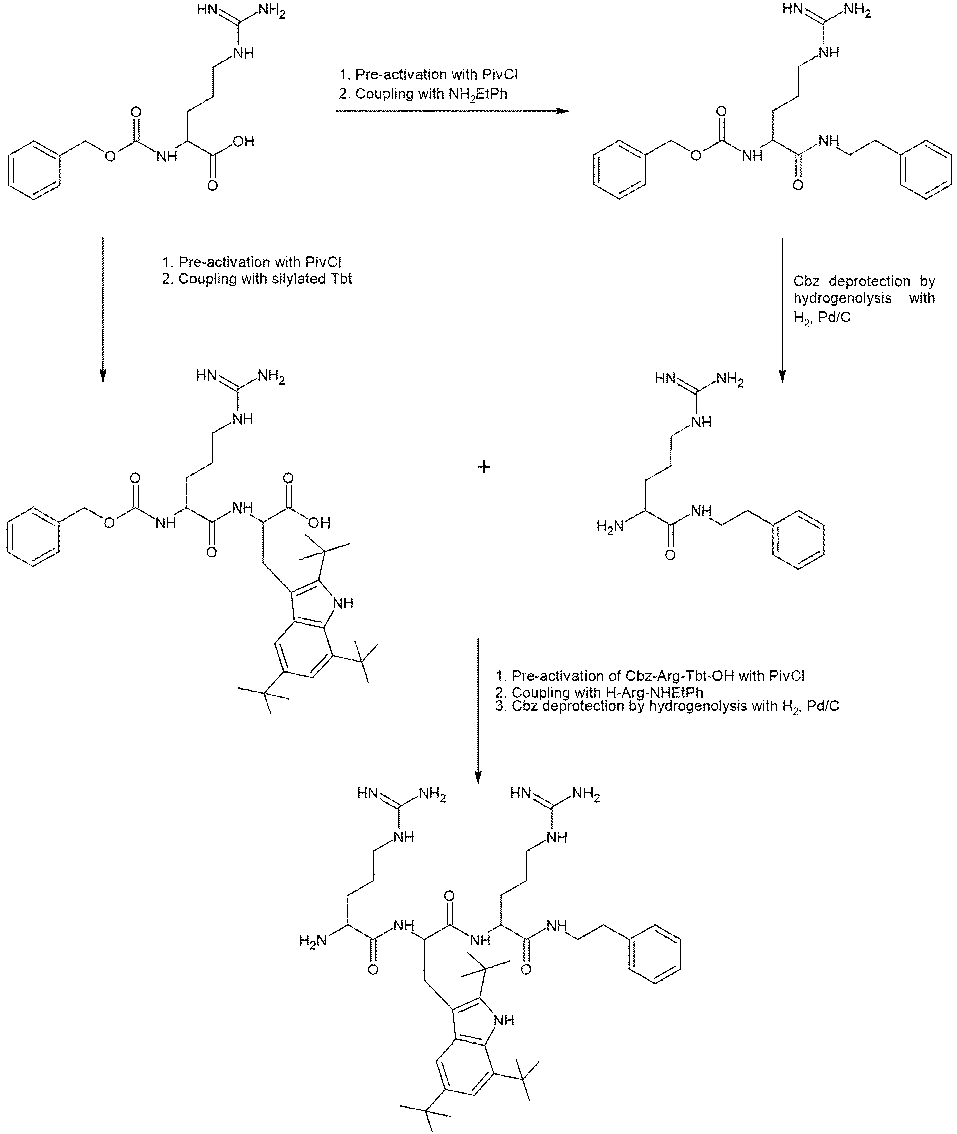

As illustrated in Scheme 1, the method of the invention may comprise steps of removing any protecting groups. For example, Cbz protecting groups may be removed by hydrogenolysis with H₂ over palladium on carbon (Pd/C).

### Examples

Materials/Methods for Examples 1-3

| Amino acid derivatives used | | |
|---|---|---|
| Fmoc-Tbt-OH Also depicted as: 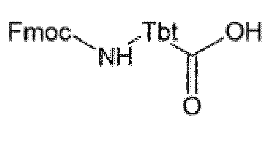 | 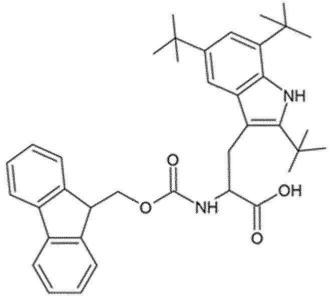 | MW= 594.35 g/mol |
| Fmoc-Aib-OH Also depicted as: 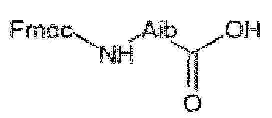 | 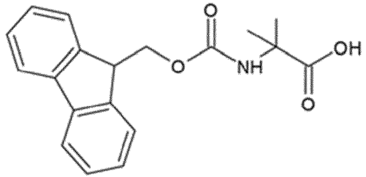 | MW= 325.13 g/mol |
| Fmoc-Tle-OH | 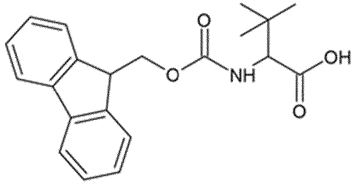 | MW= 353.16 g/mol |
| Fmoc-Ile-OH Also depicted as: 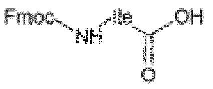 NH | 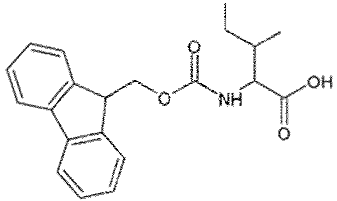 | MW= 353.16 g/mol |
| H-Arg-OMe.2HCl ** | 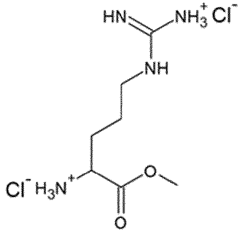 | MW= 261 g/mol |
| Cbz-Arg-OH.HCI | 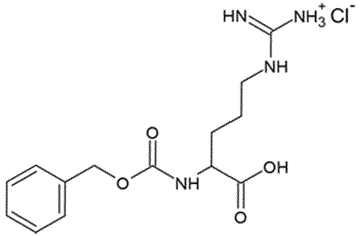 | MW= 344.80 g/mol |

| | | |
|---|---|---|
| **H-Arg-OMe is also depicted as H₂N-Arg-C(O)OMe below | | |

The amino acid derivatives are commercially available.

| Reagents used | |
|---|---|
| Diisopropylethylamine(DIPEA) | MW= 129 g/mole; d= 0.76 g/mL |
| O-(Benzotriazol-1-yl)-N,N,N'N' -tetramethyluronium hexafluorophospate (HBTU) | MW= 379 g/mole |
| Pivaloyl chloride (Piv-Cl) g/mL | MW= 121 g/mole; d= 0.98 |
| Oxalyl chloride (Oxa-Cl) g/mL | MW= 127 g/mole; d= 1.50 |
| Cyanuric chloride (Cya-Cl) g/mL | MW= 184 g/mole; d= 1.32 |
| Isobutyl chloroformate (IBCF) g/mL | MW= 137 g/mole; d= 1.05 |

| | | |
|---|---|---|
| Solvents used | | |
| Dichloromethane sieves | (DCM) | Anhydrous, kept on 3Å molecular |
| Dimethylformamide sieves | (DMF) | Anhydrous, kept on 3Å molecular |
| Methanol sieves | (MeOH) | Anhydrous, kept on 3Å molecular |

| |
|---|
| MONITORING OF REACTIONS. |

725 µL solv-B was transferred to an UPLC vial. A 25 µL reaction sample was added. The analyses were done on UPLC (see setup below) and the correct MW were confirmed using ESI-MS. The injection volume before the addition of H-Arg-OMe was 3 µL, after addition the injection volume was increased to 4 µL.

| **UPLC-PDA SETUP** | |
|---|---|
| System: | Waters Acquity H-class UPLC |
| Column: | Acquity UPLC BEH C18, 2.1 x 50 mm, with 1.7µm particles |
| Detection: | PDA (210-500 nm) |
| Mobile phases: Acetonitrile with 0.1% TFA | (Solv-A) MilliQ water with 0.1% TFA; (Solv-B) |
| Flow rate: | 0.6 mL/min |

### Gradients:

| **Gradient for Fmoc-Tbt** | | |
|---|---|---|
| **Time (min)** | **A (%)** | **B (%)** |
| 0.0 | 50 | 50 |
| 0.5 | 50 | 50 |
| 5.5 | 0 | 100 |
| 8.0 | 0 | 100 |
| 8.1 | 50 | 50 |
| 10.0 | 50 | 50 |

| **Gradient for Fmoc-Aib, Tle, and Ile** | | |
|---|---|---|
| **Time (min)** | **A (%)** | **B (%)** |
| 0.0 | 80 | 20 |
| 0.5 | 80 | 20 |
| 5.5 | 30 | 70 |
| 8.0 | 0 | 100 |
| 8.1 | 80 | 20 |
| 10.0 | 80 | 20 |

| **UPLC-MS SETUP** | |
|---|---|
| System: | Waters Acuity I-class UPLC |
| Column: | Acquity UPLC BEH C18, 2.1 x 50 mm, with 1.7µm particles |
| Detection: | Waters XEVO Q-ToF G2 mass spectrometer Tune method set at capillary 0.6 kV, sampling cone 30 V, source temperature 130 °C, desolvation temperature 450 °C, cone gas flow 10.0 L/h and desolvation gas flow 800.0 L/h |
| Mobile phases: | (Solv-A) MilliQ water with 0.1% FA; (Solv-B) Acetonitrile with 0.1% FA |
| Flow rate: | 0.5 mL/min |

### Example 1 (Comparative)

### 1.1 Activation of compound comprising a moderately sterically hindered amino acid 2-aminoisobutyric acid (Aib) using pivaloyl chloride

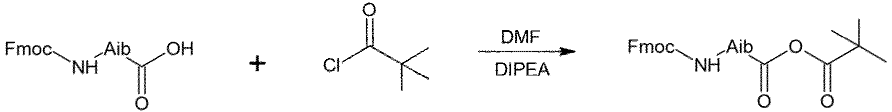

### Procedure

(i) In a small (10 mL) glass vial 3.25 mg Fmoc-Aib-OH (10 µmole) was weighed out and dissolved in 1.5 mL anhydrous DMF and 3.4 µL DIPEA (20 µmole, 2eq) was added.
(ii) The reaction was started by adding 2.5 µL Piv-Cl (20 µmole, 2eq).
(ii) Every 10 minutes a small sample was taken for UPLC-PDA analysis.
(iv) The total reaction time was 20 min.

### Result

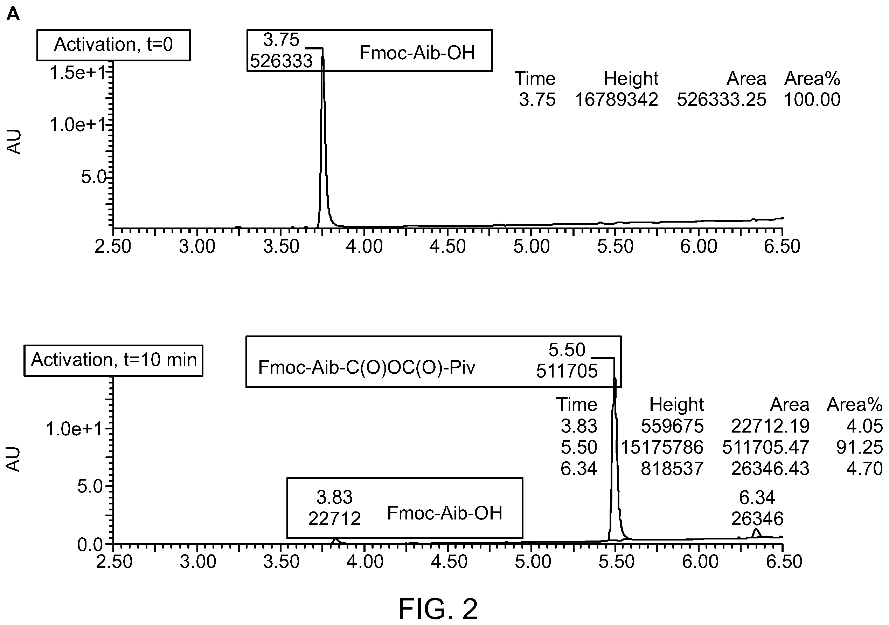

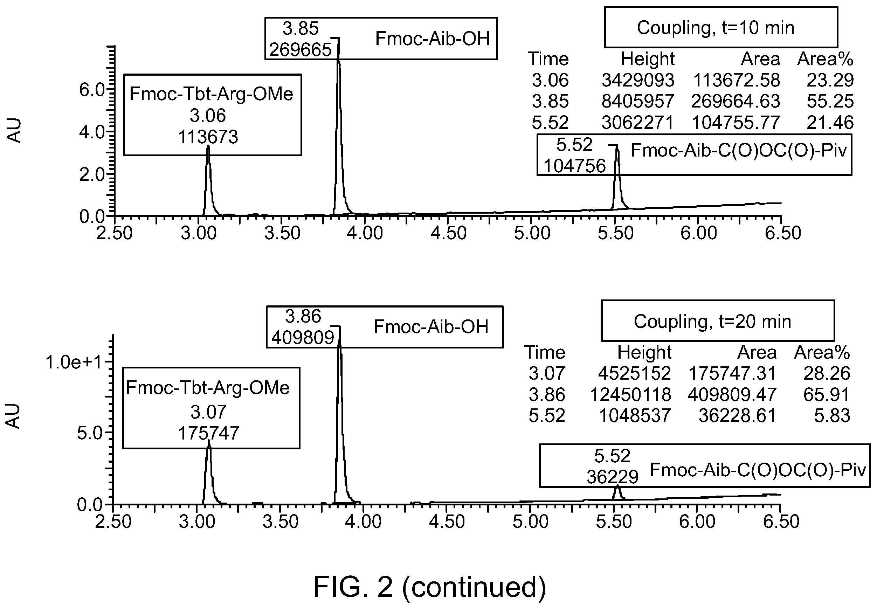

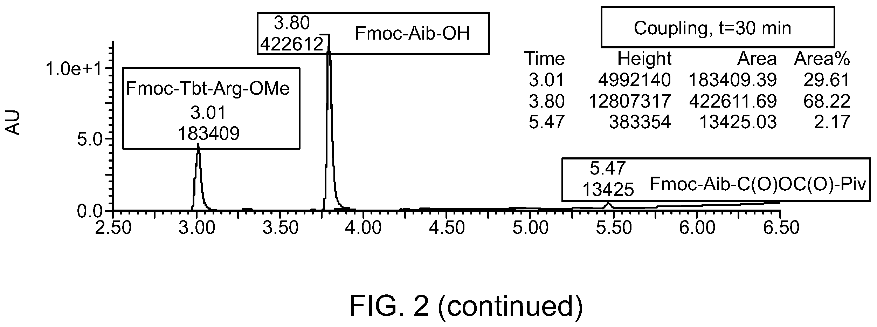

Preactivation was completed in < 10 min. A small amount (ca. 5%) of, presumably, a symmetrical anhydride of Fmoc-Aib-OH was formed, see Figure 2A.

### 1.2 Coupling reaction of Fmoc-Aib-C(O)OC(O)-Piv with H-Arg-OMe.2HCl

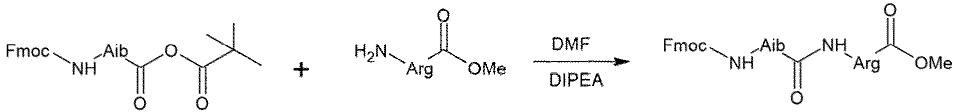

### Procedure

(i) In a small (10 mL) glass vial, 78 mg H-Arg-OMe.2HCl (300 µmole) was weighed out and dissolved in 5 mL anhydrous MeOH and 51 µL DIPEA (300 µmole, 1 eq) added.
(ii) To the pre-activation solution from reaction 1.1, 500 µL of the H-Arg-OMe.2HCl (30 µmole, 3eq) solution was added.
(iii) Every 10 minutes a small sample was taken for UPLC-PDA analysis. At the completion of the reaction, the reaction mixture was analyzed using UPLC-MS.

### Result

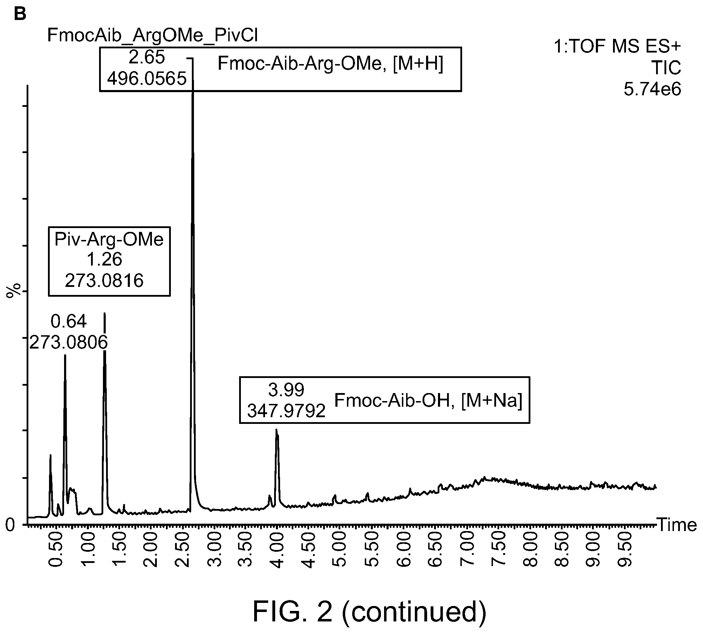

The coupling reaction is slow and is only completed after 30 min. A significant amount of unwanted side reaction of the mixed anhydride intermediate was observed, resulting in only 20% of the desired Fmoc-Aib-Arg-OMe forming, see Figure 2A and B.

### 1.3 Activation of compound comprising a moderately sterically hindered amino acid α-tert-butylglycine (Tle) using pivaloyl chloride

### Procedure

(i) In a small (10 mL) glass vial 3.53 mg Fmoc-Tle-OH (10 µmole) was weighed out and dissolved in 1.5 mL anhydrous DMF and 3.4 µL DIPEA (20 µmole, 2eq) added.
(ii) The reaction was started by adding 2.5 µL Piv-Cl (20 µmole, 2eq).
(ii) Every 10 minutes a small sample was taken for UPLC-PDA analysis.
(iv) The total reaction time was 20 min.

### Result

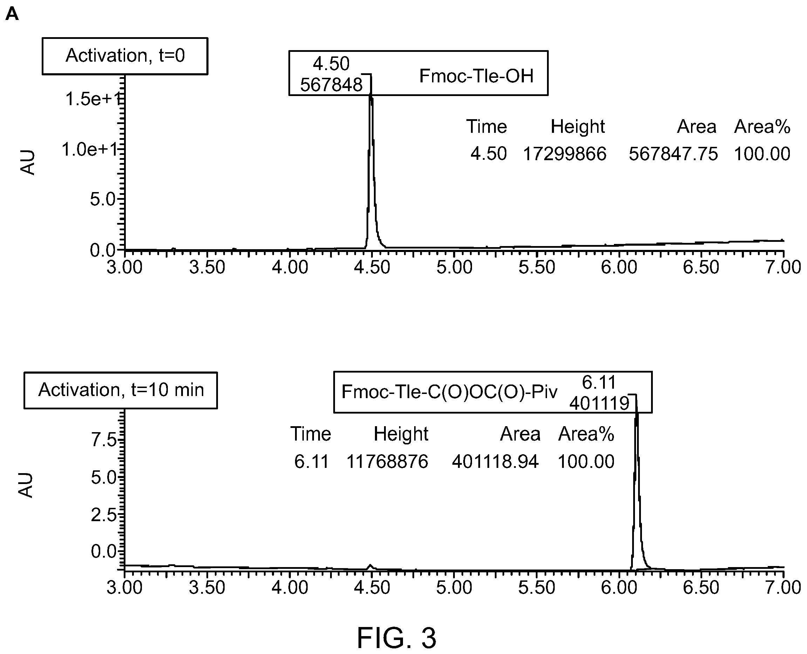

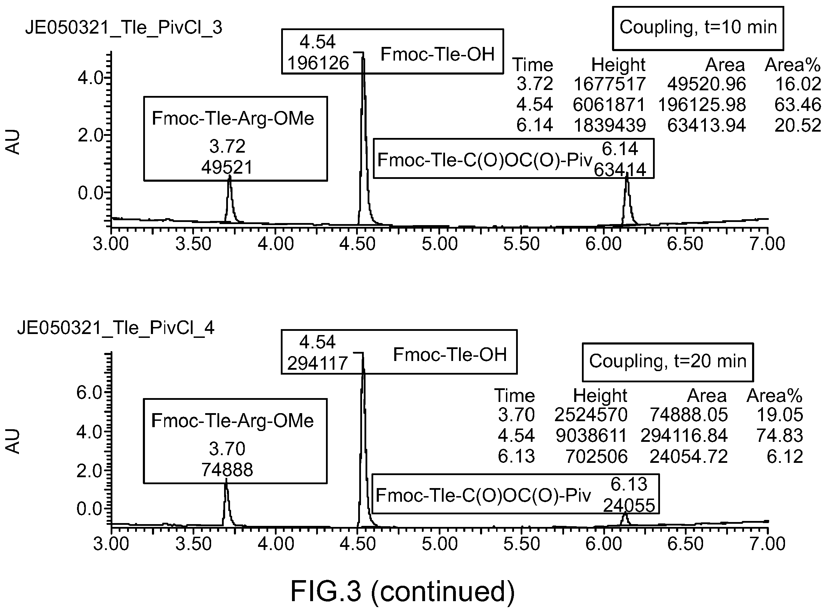

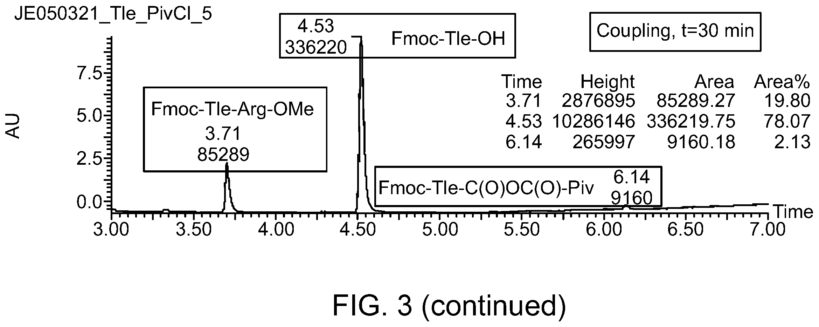

Preactivation is completed in < 10 min. No symmetrical anhydride of Fmoc-Tle-OH was observed, see Figure 3A.

### 1.4 Coupling reaction of Fmoc-Tle-C(O)OC(O)-Piv with H-Arg-OMe.2HCl

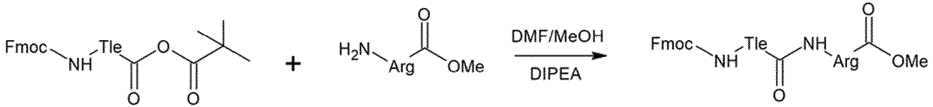

### Procedure

(i) In a small (10 mL) glass vial, 78 mg H-Arg-OMe.2HCl (300 µmole) was weighed out and dissolved in 5 mL anhydrous MeOH and 51 µL DIPEA (300 µmole, 1 eq) added.
(ii) To the pre-activation solution from reaction 1.3, 500 µL of the H-Arg-OMe.2HCl (30 µmole, 3eq) solution was added.
(iii) Every 10 minutes a small sample was taken for UPLC-PDA analysis. At the completion of the reaction, the reaction mixture was analyzed using UPLC-MS.

### Result

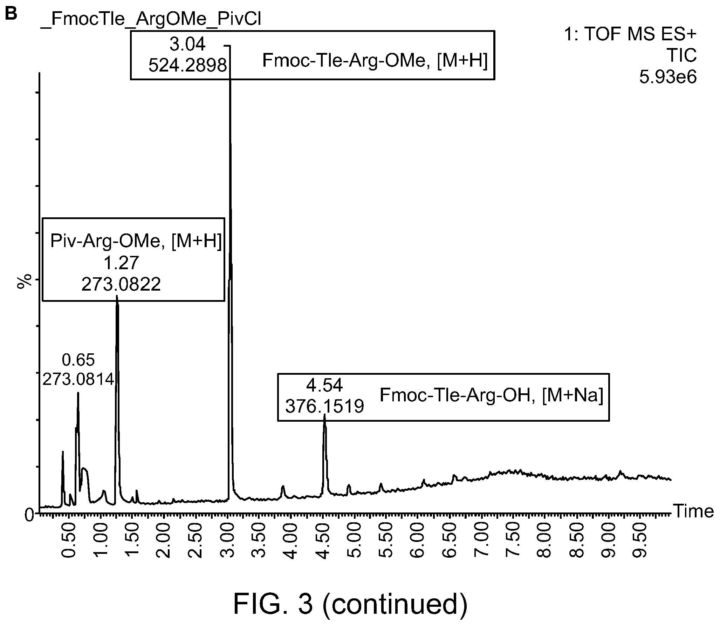

The coupling reaction is slow and is only completed after 30 min. A significant amount of unwanted side reaction of the mixed anhydride intermediate was observed, resulting in only 40% of the desired Fmoc-Tle-Arg-OMe forming, see Figure 3B.

### 1.5 Activation of compound comprising a moderately sterically hindered amino acid isoleucine (Ile) using pivaloyl chloride

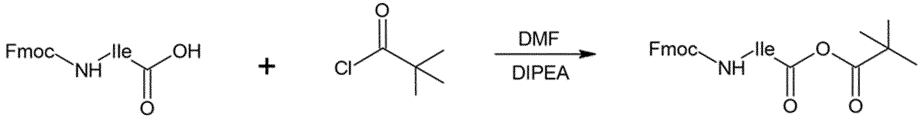

### Procedure

(i) In a small (10 mL) glass vial 3.53 mg Fmoc-Ile-OH (10 µmole) was weighed out and dissolved in 1.5 mL anhydrous DMF and 3.4 µL DIPEA (20 µmole, 2eq) added.
(ii) The reaction was started by adding 2.5 µL Piv-Cl (20 µmole, 2eq).
(ii) Every 10 minutes a small sample was taken for UPLC-PDA analysis.
(iv) The total reaction time was 20 min.

### Result

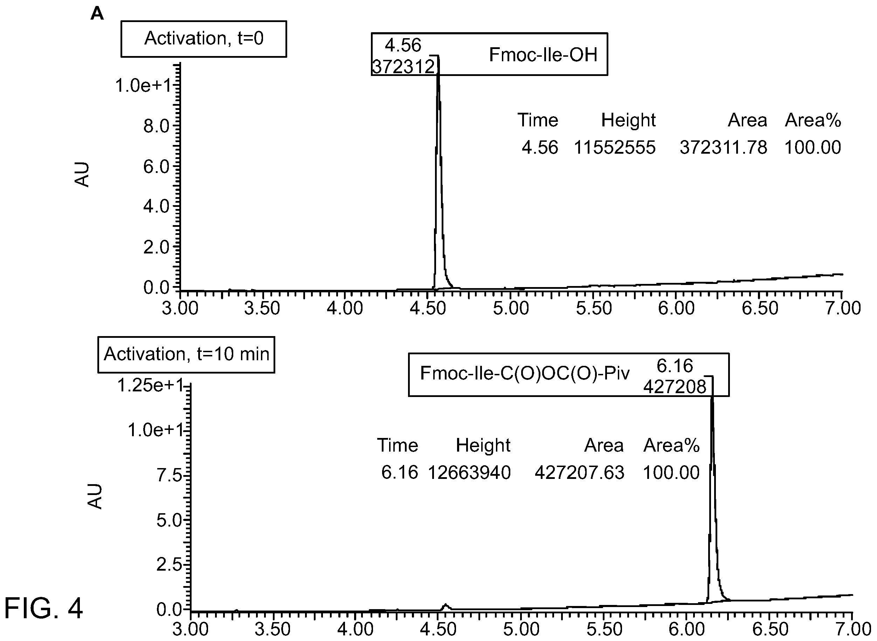

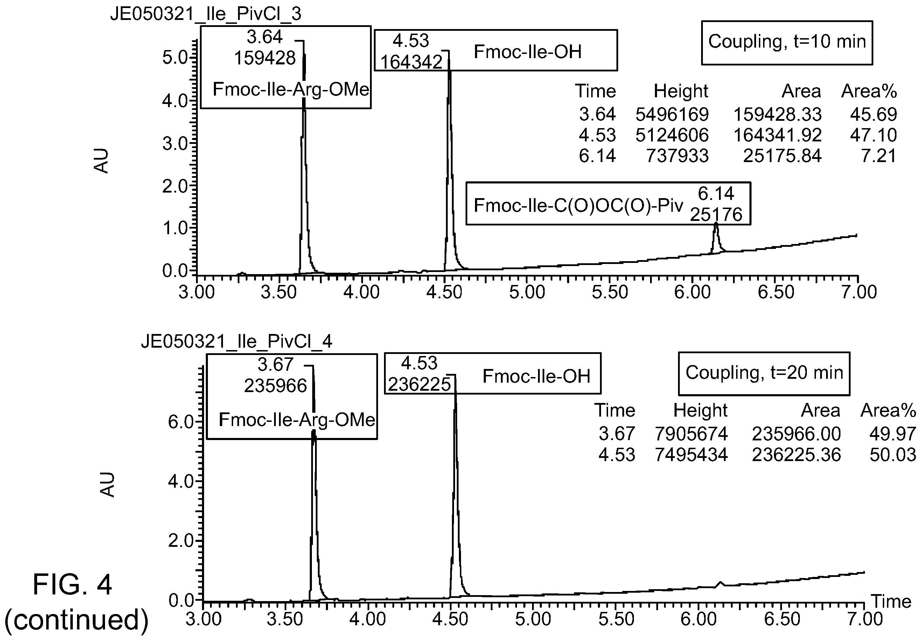

Preactivation is completed in < 10 min. No symmetrical anhydride of Fmoc-Ile-OH was observed, see Figure 4A.

### 1.6 Coupling reaction of Fmoc-Ile-C(O)OC(O)-Piv with H-Arg-OMe.2HCl

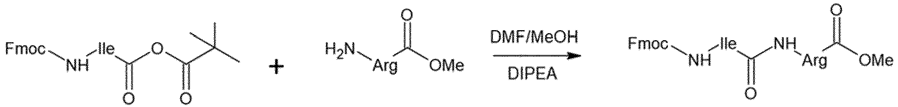

### Procedure

(i) In a small (10 mL) glass vial, 78 mg H-Arg-OMe.2HCl (300 µmole) was weighed out and dissolved in 5 mL anhydrous MeOH and 51 µL DIPEA (300 µmole, 1 eq) added.
(ii) To the pre-activation solution from reaction 1.5, 500 µL of the H-Arg-OMe.2HCl (30 µmole, 3eq) solution was added.
(iii) Every 10 minutes a small sample was taken for UPLC-PDA analysis. At the completion of the reaction, the reaction mixture was analyzed using UPLC-MS.

### Result

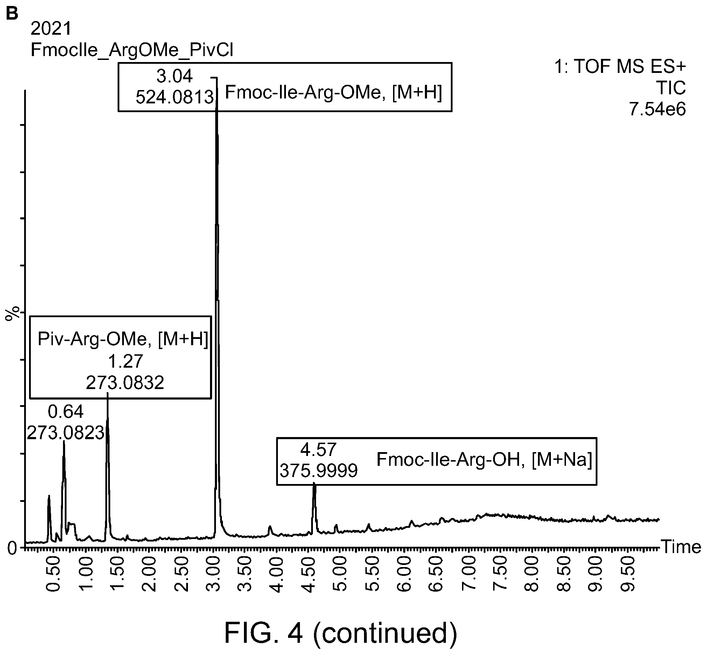

The coupling reaction is relatively slow and is completed after 20 min. A significant amount of unwanted side reaction of the mixed anhydride intermediate was observed, resulting in only 37% of the desired Fmoc-Ile-Arg-OMe forming, see Figure 4A and 4B.

As shown in Example 1, a significant amount of unwanted side reaction of the mixed anhydride intermediates at the "wrong" carbonyl was observed for moderately sterically hindered amino acids containing Aib, Tle or Ile after activation with pivaloyl chloride.

### Example 2

### 2.1 Activation of amino acid comprising tri-tert-butyl-tryptophan (Tbt) using pivaloyl chloride

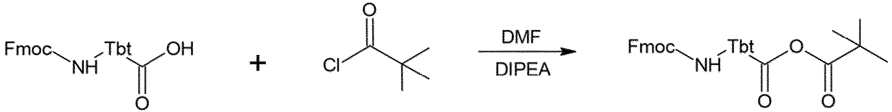

### Procedure

(i) In a small (10 mL) glass vial 5.95 mg Fmoc-Tbt-OH (10 µmole) was weighed out and dissolved in 1.5 mL anhydrous DMF and 3.4 µL DIPEA (20 µmole, 2eq) added.
(ii) The reaction was started by adding 2.5 µL Piv-Cl (20 µmole, 2eq).
(ii) Every 10 minutes a small sample was taken for UPLC-PDA analysis.
(iv) The total reaction time was 20 min.

### Results

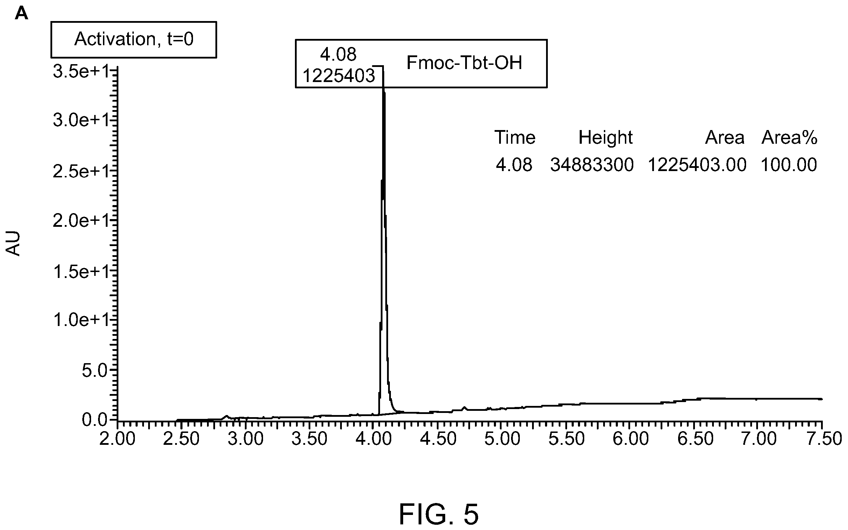

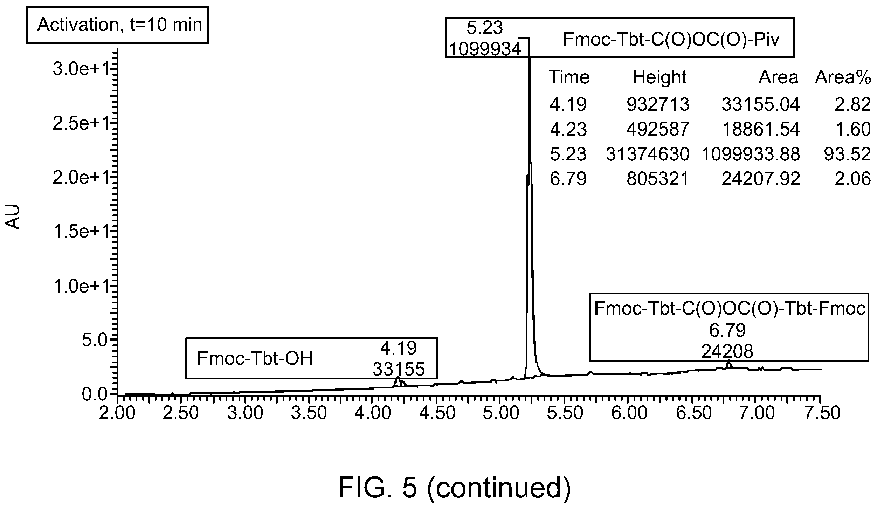

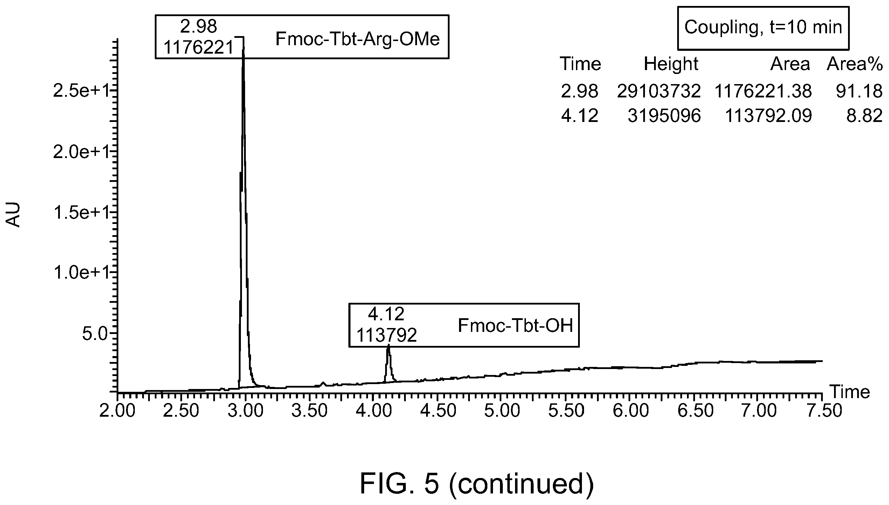

Preactivation is completed in less than 10 min, see Figure 5A. The formed Fmoc-Tbt-C(O)OC(O)Piv is stable and no degradation was observed during the 60 min pre-activation period (results not shown). A small amount (ca. 2%) of, presumably, a symmetrical anhydride of Fmoc-Tbt-OH was formed. This reaction was also possible when using DCM as solvent (results not shown).

### 2.2 Coupling reaction of Fmoc-Tbt-C(O)OC(O)-Piv with H-Arg-OMe.2HCl

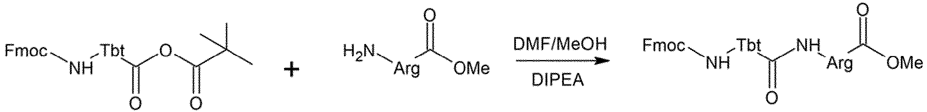

### Procedure

(i) In a small (10 mL) glass vial, 78 mg H-Arg-OMe.2HCl (300 µmole) was weighed out and dissolved in 5 mL anhydrous MeOH and 51 µL DIPEA (300 µmole, 1 eq) added.
(ii) To the pre-activation solution from reaction 2.1, 500 µL of the H-Arg-OMe.2HCl (30 µmole, 3eq) solution was added.
(iii) Every 10 minutes a small sample was taken for UPLC-PDA analysis. At the completion of the reaction, the reaction mixture was analyzed using UPLC-MS.

### Results

The coupling reaction is unexpectedly fast and is completed < 10 min. The main product is the desired Fmoc-Tbt-Arg-OMe, but there is a small amount (< 9%) of Fmoc-Tbt-OH. This is due to the α-amine group of H-Arg-OMe attacking the wrong carbonyl of the formed mixed anhydride.

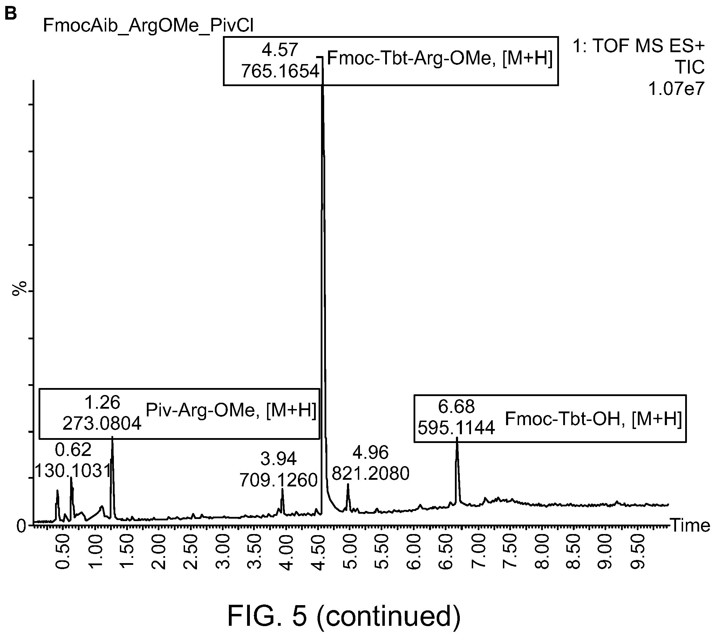

The presence of Piv-Arg-OMe was confirmed with UPLC-MS, see Figure 5B. The reason for this is most likely due to the steric hinderance effect of Tbt's bulky side chain. To ensure that the reaction was between the α-amine group of H-Arg-OMe and not the guanidine group, the reaction was repeated using Cbz-Arg-OH.HCI. No reaction was observed (results not shown).

Although a small amount of unwanted side reaction of the mixed anhydride intermediate was observed when coupling the ultra-bulky Fmoc-Tbt-OH and H-Arg-OMe.2HCl, it was significantly less than that observed for the other, less bulky, amino acids Fmoc-Aib-OH, Fmoc-Tle-OH, and Fmoc-Ile-OH.

The coupling reactions were fast, with no signs of Tbt-epimerization or unwanted acylation through the unprotected guanidine side chain.

### Example 3

### 3.1 Activation of amino acid comprising tri-tert-butyl-tryptophan (Tbt) using isobutyl chloroformate (IBCF)

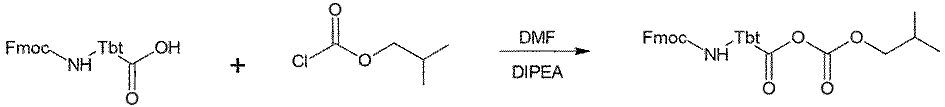

### Procedure

(i) In a small (10 mL) glass vial 5.95 mg Fmoc-Tbt-OH (10 µmole) was weighed out and dissolved in 1.5 mL anhydrous DMF and 3.4 µL DIPEA (20 µmole, 2eq) added.
(ii) The reaction was started by adding 2.6 µL IBCF (20 µmole, 2eq).
(ii) Every 10 minutes a small sample was taken for UPLC-PDA analysis.
(iv) The total reaction time was 20 min.

### Result

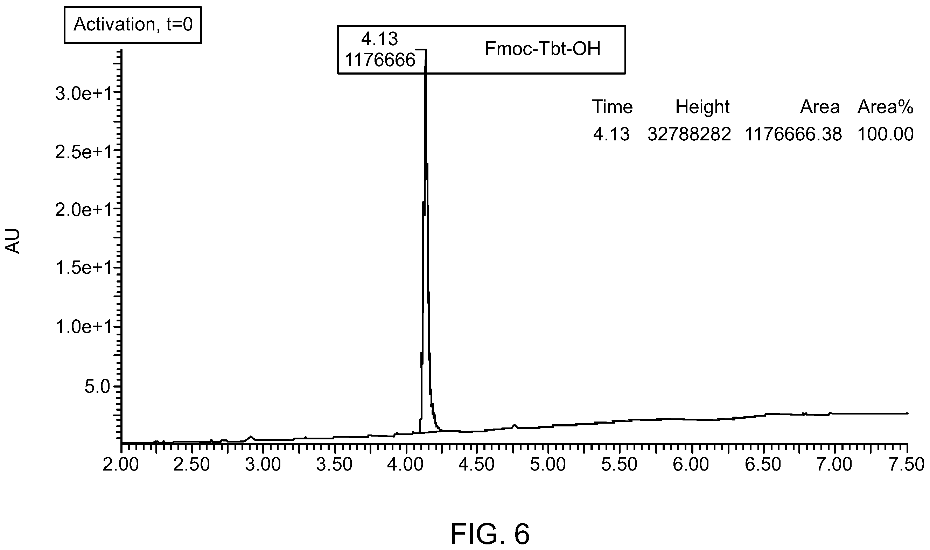

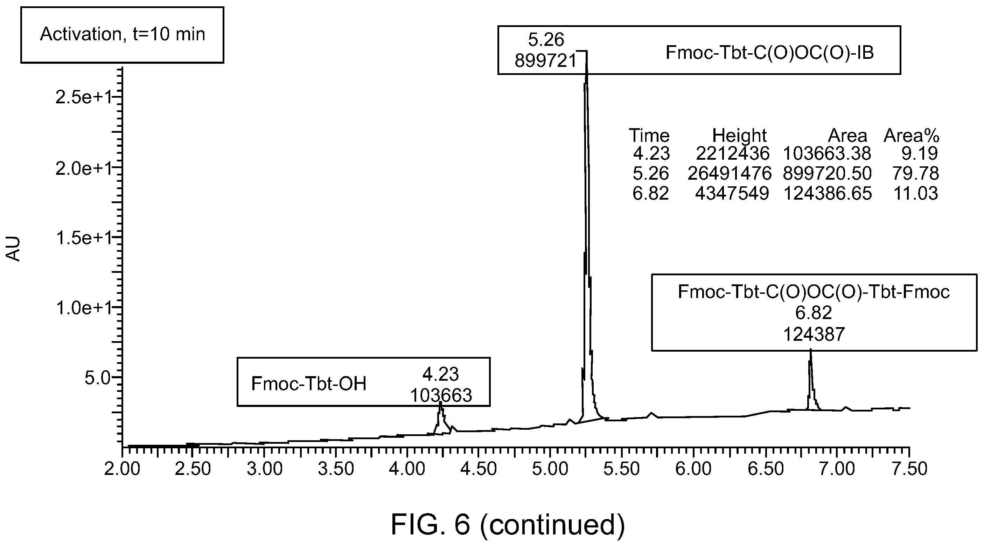

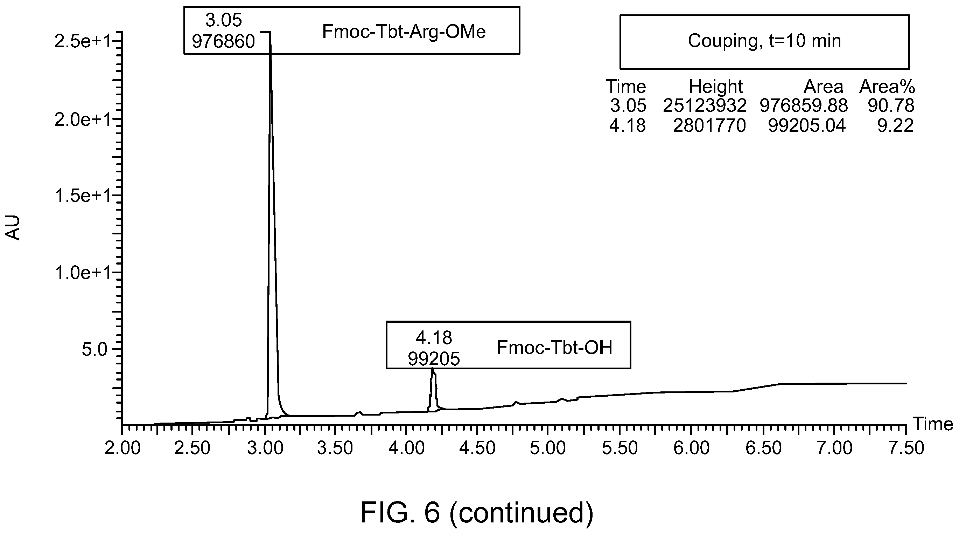

Preactivation is completed in < 10 min. A significant amount (ca. 11%) of, presumably, a symmetrical anhydride of Fmoc-Tbt-OH was formed, see Figure 6A.

### 3.2 Coupling reaction of Fmoc-Tbt-C(O)OC(O)-IB with H-Arg-OMe.2HCl

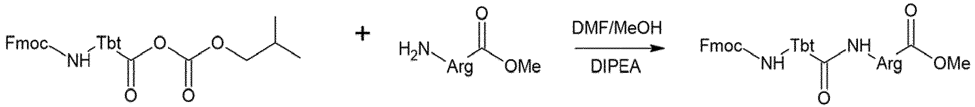

### Procedure

(i) In a small (10 mL) glass vial, 78 mg H-Arg-OMe.2HCl (300 µmole) was weighed out and dissolved in 5 mL anhydrous MeOH and 51 µL DIPEA (300 µmole, 1 eq) added.
(ii) To the pre-activation solution from reaction 3.1, 500 µL of the H-Arg-OMe.2HCl (30 µmole, 3eq) solution was added.
(iii) Every 10 minutes a small sample was taken for UPLC-PDA analysis. At the completion of the reaction, the reaction mixture was analyzed using UPLC-MS.

### Result

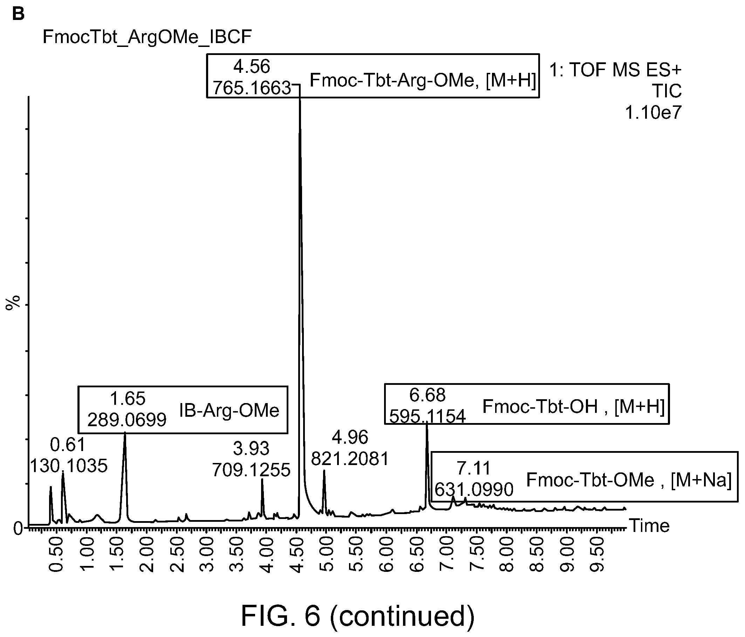

The coupling reaction is fast and is completed < 10 min. The main product is the desired Fmoc-Tbt-Arg-OMe, but there is also a small amount (ca. 9%) of Fmoc-Tbt-OH present (Figure 6B).

### Example 4

### 4.1 Preparation of intermediates Z-Arg-Tbt-OH (AMC-01) and

### H-Arg-NHEtPh (AMC-03)

Z-Arg-Tbt-OH (AMC-01) was prepared by activation of Cbz-protected arginine with IBCF followed by coupling with silylated Tbt as illustrated in the following scheme.

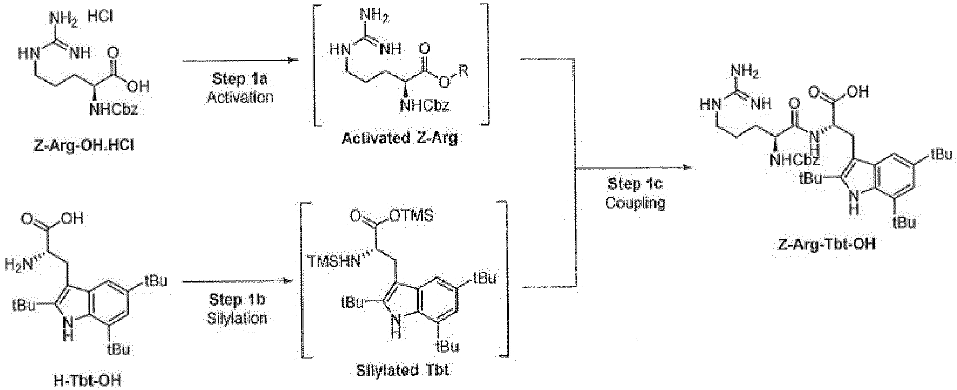

Z-Arg-NHEtPh (AMC-02) was prepared by activating commercially available Z-Arg-OH.HCI with IBCF, reacting activated Cbz-protected arginine with H₂NEtPh, and deprotection to provide H-ArgNHEtPh (AMC-03) as illustrated in the following schemes:

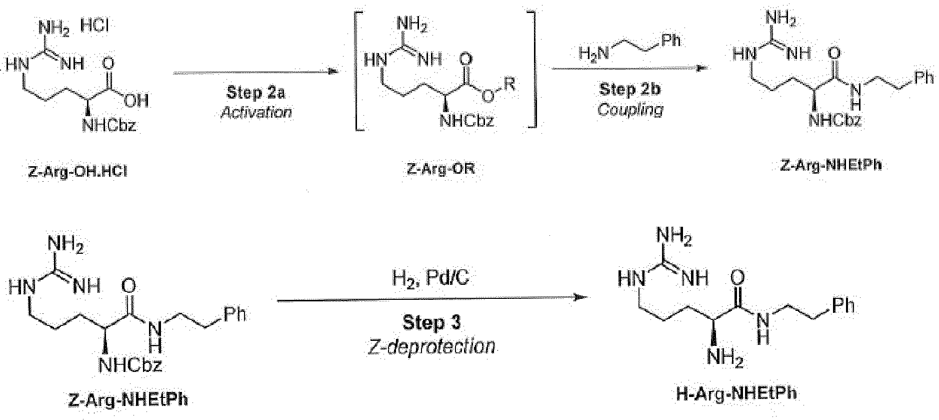

The procedure for the deprotection step (Step 3) was as follows. AMC-02 (37.20 g, 75% wt.), MeOH (550 mL) and water (130 mL) were introduced into a 2 L three neck flask. The suspension was stirred under nitrogen until all of the AMC-02 was fully soluble. Pd/C (10%wt, 50% wet, 2.27 g, 1.5 mol%) was added and the N₂ atmosphere was replaced with H₂ (using a H₂ generator, 0.7 bar). Deprotection conversion was followed by HPLC and full conversion was obtained after 3 h. Pd/C was then filtered, washed twice with MeOH/water (8/2, v/v, 2 x 75 mL). The filtrates were combined and concentrated under reduced pressure (Tbath = 55 °C, from 300 mbar to 50 mbar) up to 31 g of concentrated solution. DMA (100 mL) was added and evaporation was pursued (Tbath = 65 °C, 25 mbar) to afford AMC-03 as a colourless solution (117 mL, 115 g, 17% wt by NMR, est. 19.6 g net peptide, > 99% yield).

### 4.2 Coupling of Z-Arg-Tbt-OH (AMC-01) and Z-Arg-NHEtPh (AMC-02) to provide Z-Arg-Tbt-Arg-NHEtPh (AMC-04)

Activation of AMC-01 (1 eq) was carried out with PivCl (1.05 eq) and pyridine (1.05 eq) in a 2:1 v/v mixture of ACN/2-MeTHF at -10°C for 15 minutes.

The colourless solution of AMC-03 was acidified with 2.5 N aqueous HCl (3.15 eq vs AMC-03). AMC-03 (1.05 eq) in the form of this acidified solution was then added to the activated AMC-01, followed by DIPEA (2,10 eq vs AMC-03). The coupling reaction was conducted at -10 °C for 1 hour and progress of the reaction was followed by HPLC.

The parameters for the HPLC method are set out below:

| | | | | | | |
|---|---|---|---|---|---|---|
| Column | Waters XSelect CSH C18 XP, 130 A, 2.5 pm, 3 x 75 mm | | | | | |
| Oven Temperature | 40 °C | | | | | |
| Wavelength | 220 nm | | | | | |
| Mobile Phases | A : 0.1% TFA in water | | | | | |
| | B: 0.1% TFA in ACN | | | | | |
| Flow Rate | 1 mL/min | | | | | |
| Gradient | Time (min) | 0 | 7.5 | 8.1 | 8.2 | 10.7 |
| | % A | 98 | 0 | 0 | 98 | 98 |
| | %B | 2 | 100 | 100 | 2 | 2 |

### Result

The main product is the desired Z-Arg-Tbt-Arg-NHEtPh and only a small amount of AMC-01 (9 %) was present.

## Claims

1. A method for making a target peptide comprising reacting a mixed anhydride compound of Formula (I) with a second moiety which is an amino acid or peptide;
wherein Formula (I) has the structure: 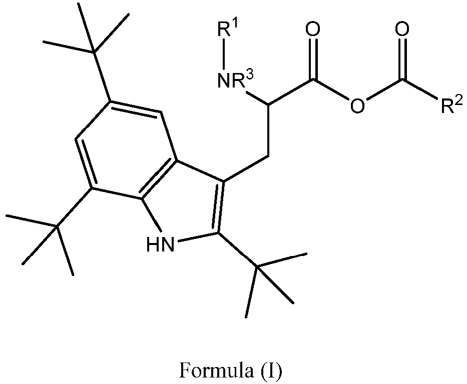
wherein R¹ is a protecting group, a peptide or an amino acid;
wherein R² is *tert-*butyl, isobutoxy, *tert*-butoxy, isobutyl, isopropoxy, isopropyl, or ethoxy; and
wherein R³ is H or an alkylsilyl group.

2. The method of claim 1 comprising preparing the mixed anhydride compound of Formula (I) by reacting a first moiety of Formula (II) with an activator of Formula (III) in the presence of a base;
wherein Formula (II) has the structure: 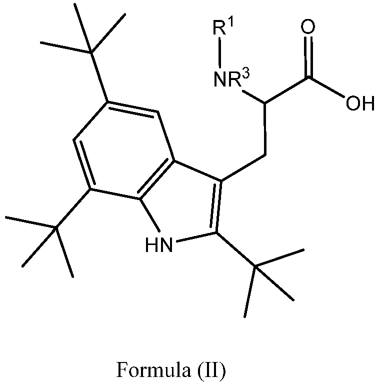 wherein R¹ is a protecting group, a peptide or an amino acid and R³ is H or an alkylsilyl group;
and wherein Formula (III) has the structure: 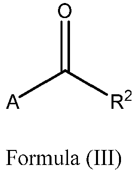
wherein R² is *tert*-butyl, isobutoxy, *tert*-butoxy, isobutyl, isopropoxy, isopropyl, or ethoxy; and
A is a halogen.

3. The method of claim 1 or claim 2, wherein
(A) R³ is H; and/or
(B) R¹ is a protecting group selected from benzyloxycarbonyl (Cbz), tert-butoxycarbonyl (Boc), 4-methoxy-2,3,6-trimethylbenzene sulphonyl (Mtr), 9-fluorenylmethoxy-carbonyl (Fmoc) and 2,2,2-trichloroethoxycarbonyl (Troc); a peptide or an amino acid; and/or
(C) R¹ is a peptide or an amino acid, preferably wherein R¹ is an amino acid.

4. The method of any one of the preceding claims, wherein:
(A) R² is *tert*-butyl or isobutoxy, preferably *tert*-butyl; or
(B) R² is *tert*-butyl, isobutyl, or isopropyl.

5. The method of any one of the preceding claims wherein the R¹ amino acid is a cationic amino acid AA₁, preferably wherein AA₁ is arginine.

6. The method of any one of claims 2-5, further comprising preparing a compound of Formula (II), preferably wherein R¹ is a peptide or an amino acid and the method comprises coupling said peptide or amino acid to a tri-tert-butyl-tryptophan (Tbt) residue.

7. The method of any one of the preceding claims wherein the second moiety comprises one or more protecting groups and/or a C-terminal capping group.

8. The method of claim 7, wherein the C-terminal capping group is of formula -X-Y-Z, wherein:
X is a N atom, which may be substituted by a branched or unbranched C₁-C₁₀ alkyl or
aryl group, and this alkyl or aryl group may incorporate up to 2 heteroatoms selected from N, O and S;
Y represents a group selected from -Rₐ-R_{b}-, -Rₐ-R_{b}-R_{b}- and -R_{b}-R_{b}-Rₐ- wherein
Rₐ is C, O, S or N, and
R_{b} is C; each of Rₐ and R_{b} may be substituted by C₁-C₄ alkyl groups or unsubstituted; and
Z is a group comprising 1 to 3 cyclic groups each of 5 or 6 non-hydrogen atoms, 2 or more of the cyclic groups may be fused and one or more of the cyclic groups may be substituted; the Z moiety incorporates a maximum of 15 non-hydrogen atoms; and wherein
the bond between Y and Z is a covalent bond between Rₐ or R_{b} of Y and a non-hydrogen atom of one of the cyclic groups of Z.

9. The method of any one of the preceding claims, wherein the second moiety is an amino acid comprising AA₃, or a peptide comprising AA₃ as the N-terminal amino acid, wherein AA₃ is a cationic amino acid.

10. The method of any one of the preceding claims, wherein the second moiety is a compound of Formula (IV)
AA₃-X-Y-Z (IV)
wherein:
AA₃ is a cationic amino acid;
X is a N atom, which may be substituted by a branched or unbranched C₁-C₁₀ alkyl or aryl group, and this alkyl or aryl group may incorporate up to 2 heteroatoms selected from N, O and S;
Y represents a group selected from -Rₐ-R_{b}-, -Rₐ-R_{b}-R_{b}- and -R_{b}-R_{b}-Rₐ- wherein
Rₐ is C, O, S or N, and
R_{b} is C; each of Rₐ and R_{b} may be substituted by C₁-C₄ alkyl groups or unsubstituted; and
Z is a group comprising 1 to 3 cyclic groups each of 5 or 6 non-hydrogen atoms, 2 or more of the cyclic groups may be fused and one or more of the cyclic groups may be substituted; the Z moiety incorporates a maximum of 15 non-hydrogen atoms; and wherein
the bond between Y and Z is a covalent bond between Rₐ or R_{b} of Y and a non-hydrogen atom of one of the cyclic groups of Z.

11. The method of claim 9 or claim 10, wherein AA₃ is lysine and/or arginine, preferably arginine.

12. The method of any one of claims 8-11 wherein:
(A) X is unsubstituted; and/or
(B) Rₐ is C; and/or
(C) Y is -Rₐ-R_{b}- and unsubstituted, preferably wherein Y is -CH₂-CH₂-; and/or
(D) Z is phenyl.

13. The method of any one of the preceding claims, wherein the target peptide has the structure:

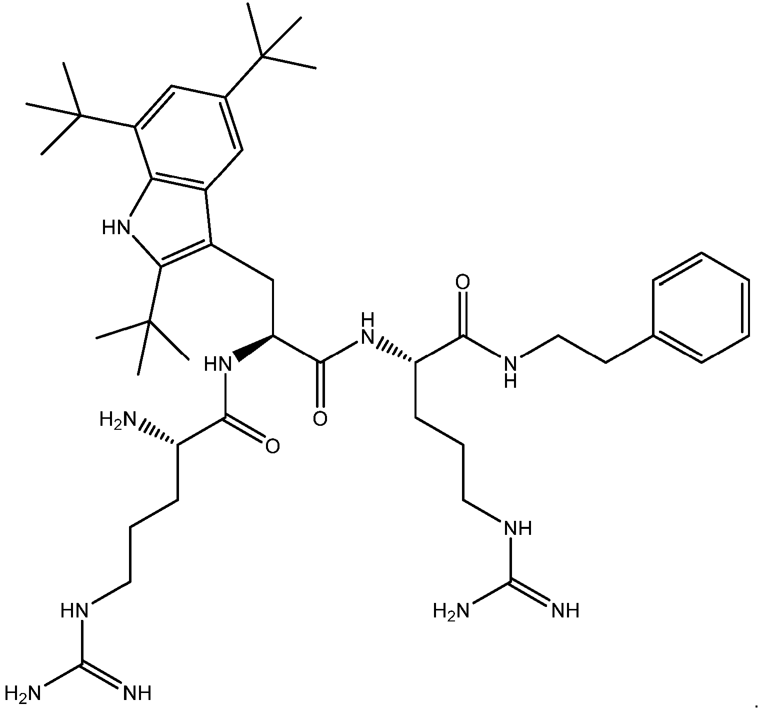

14. The method of any one of the preceding claims, wherein the amino acid or peptide of the second moiety is provided in the form of a salt.

15. A compound of Formula (I) as defined in any one of claims 1 or 3-5.

## Patentansprüche

1. Herstellungsverfahren eines Zielpeptids, umfassend die Reaktion einer Mischsäureanhydridverbindung der Formel (I) mit einem zweiten Molekülteil, der eine Aminosäure oder ein Peptid ist;
wobei die Formel (I) die Struktur aufweist: 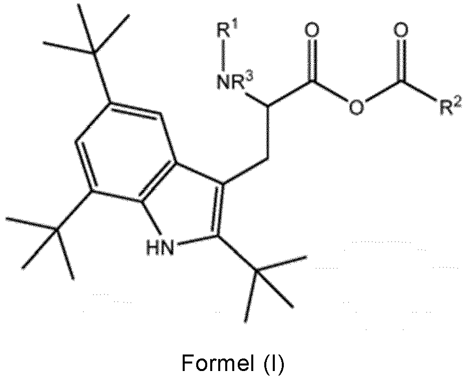
wobei R¹ eine Schutzgruppe, ein Peptid oder eine Aminosäure ist;
wobei R² *tert*-Butyl, Isobutoxy, *tert*-Butoxy, Isobutyl, Isopropoxy, Isopropyl oder Ethoxy ist; und
wobei R³ H oder eine Alkylsilylgruppe ist.

2. Verfahren nach Anspruch 1, umfassend die Herstellung der Mischsäureanhydridverbindung der Formel (I) durch Reaktion eines ersten Molekülteils der Formel (II) mit einem Aktivator der Formel (III) in Gegenwart einer Base;
wobei die Formel (II) die Struktur aufweist: 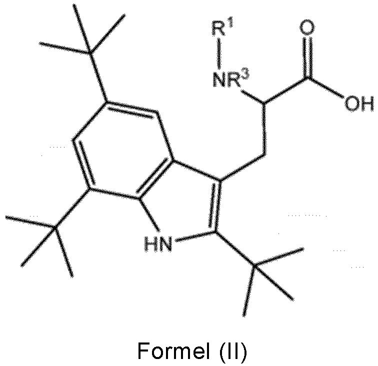 wobei R¹ eine Schutzgruppe, ein Peptid oder eine Aminosäure ist und R³ H oder eine Alkylsilylgruppe ist;
und wobei die Formel (III) die Struktur aufweist: 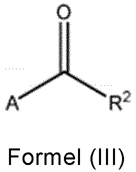
wobei R² *tert-*Butyl, Isobutoxy, *tert-*Butoxy, Isobutyl, Isopropoxy, Isopropyl oder Ethoxy ist; und
A ein Halogen ist.

3. Verfahren nach Anspruch 1 oder Anspruch 2, wobei
(A) R³ H ist; und/oder
(B) R¹ eine Schutzgruppe ist, die ausgewählt ist aus Benzyloxycarbonyl (Cbz), tert-Butoxycarbonyl
(Boc) 4-Methoxy-2,3,6-trimethylbenzolsulphonyl (Mtr), 9-Fluorenylmethoxycarbonyl
(Fmoc) und 2,2,2-Trichloroethoxycarbonyl (Troc); einem Peptid oder einer Aminosäure; und/oder
(C) R¹ ein Peptid oder eine Aminosäure ist, vorzugsweise wobei R¹ eine Aminosäure ist.

4. Verfahren nach einem der vorstehenden Ansprüche, wobei:
(A) R² *tert-*Butyl oder Isobutylo, vorzugsweise *tert-*Butyl ist; oder
(B) R² *tert-*Butyl*,* Isobutyl oder Isopropyl ist.

5. Verfahren nach einem der vorstehenden Ansprüche wobei die R¹-Aminosäure eine kationische Aminosäure AA₁ ist, vorzugsweise wobei AA₁ Arginin ist.

6. Verfahren nach einem der Ansprüche 2-5, weiter umfassend die Herstellung einer Verbindung der Formel (II), vorzugsweise wobei R¹ ein Peptid oder eine Aminosäure ist und wobei das Verfahren die Kopplung des Peptids oder der Aminosäure an einen Tri-tert-butyltryptophan (Tbt)-Rest umfasst.

7. Verfahren nach einem der vorstehenden Ansprüche wobei der zweite Molekülteil eine oder mehrere Schutzgruppen und/oder eine C-terminale Kappungsgruppe umfasst.

8. Verfahren nach Anspruch 7, wobei die C-terminale Kappungsgruppe eine Formel -X-y-Z ist, wobei:
X ein N-Atom ist, das durch eine verzweigte oder unverzweigte C₁-C₁₀-Alkyl- oder Arylgruppe substituiert sein kann, und diese Alkyl- oder Arylgruppe bis zu 2 Heteroatome, die ausgewählt sind aus N, O und S, enthalten kann;
Y eine Gruppe repräsentiert, die ausgewählt ist aus -Rₐ₋R_{b}-, -Rₐ₋R_{b}-R_{b}- und -R_{b}-R_{b}-Rₐ₋ wobei
Rₐ C, O, S oder N ist, und
R_{b} C ist; jedes von Rₐ und R_{b} durch C₁-C₄-Alkylgruppen substituiert oder unsubstituiert sein kann; und
Z eine Gruppe ist, die 1 bis 3 cyclische Gruppen mit jeweils 5 oder 6 Nicht-Wasserstoffatomen umfasst, wobei 2 oder mehr der cyclischen Gruppen kondensiert sein können und eine oder mehrere der cyclischen Gruppen substituiert sein können; wobei der Z-Molekülteil maximal 15 Nicht-Wasserstoffatome enthält; und wobei
die Bindung zwischen Y und Z eine kovalente Bindung zwischen Rₐ oder R_{b} von Y und einem Nicht-Wasserstoffatom einer der cyclischen Gruppen von Z ist.

9. Verfahren nach einem der vorstehenden Ansprüche, wobei der zweite Molekülteil eine Aminosäure, die AA₃ umfasst, oder ein Peptid, das AA₃ als N-terminale Aminosäure umfasst, ist, wobei AA₃ eine kationische Aminosäure ist.

10. Verfahren nach einem der vorstehenden Ansprüche, wobei der zweite Molekülteil eine Verbindung der Formel (IV) ist
AA₃-X-y-Z (IV)
wobei:
AA₃ eine kationische Aminosäure ist;
X ein N-Atom ist, das durch eine verzweigte oder unverzweigte C₁-C₁₀-Alkyl- oder Arylgruppe substituiert sein kann, und diese Alkyl- oder Arylgruppe bis zu 2 Heteroatome, die ausgewählt sind aus N, O und S, enthalten kann;
Y eine Gruppe repräsentiert, die ausgewählt ist aus -Rₐ₋R_{b}-, -Rₐ₋R_{b}-R_{b}- und -R_{b}-R_{b}-Rₐ₋ wobei
Rₐ C, O, S oder N ist, und
R_{b} C ist; jedes von Rₐ und R_{b} durch C₁-C₄-Alkylgruppen substituiert oder unsubstituiert sein kann; und
Z eine Gruppe ist, die 1 bis 3 cyclische Gruppen mit jeweils 5 oder 6 Nicht-Wasserstoffatomen umfasst, wobei 2 oder mehr der cyclischen Gruppen kondensiert sein können und eine oder mehrere der cyclischen Gruppen substituiert sein können; wobei der Z-Molekülteil maximal 15 Nicht-Wasserstoffatome enthält; und wobei
die Bindung zwischen Y und Z eine kovalente Bindung zwischen Rₐ oder R_{b} von Y und einem Nicht-Wasserstoffatom einer der cyclischen Gruppen von Z ist.

11. Verfahren nach Anspruch 9 oder Anspruch 10, wobei AA₃ Lysin und/oder Arginin, vorzugsweise Arginin, ist.

12. Verfahren nach einem der Ansprüche 8-11, wobei:
(A) X unsubstituiert ist; und/oder
(B) Rₐ C ist; und/oder
(C) Y -Rₐ₋R_{b}- und unsubstituiert ist, vorzugsweise wobei Y -CH₂-CH₂- ist; und/oder
(D) Z Phenyl ist.

13. Verfahren nach einem der vorstehenden Ansprüche, wobei das Zielpeptid die Struktur aufweist:

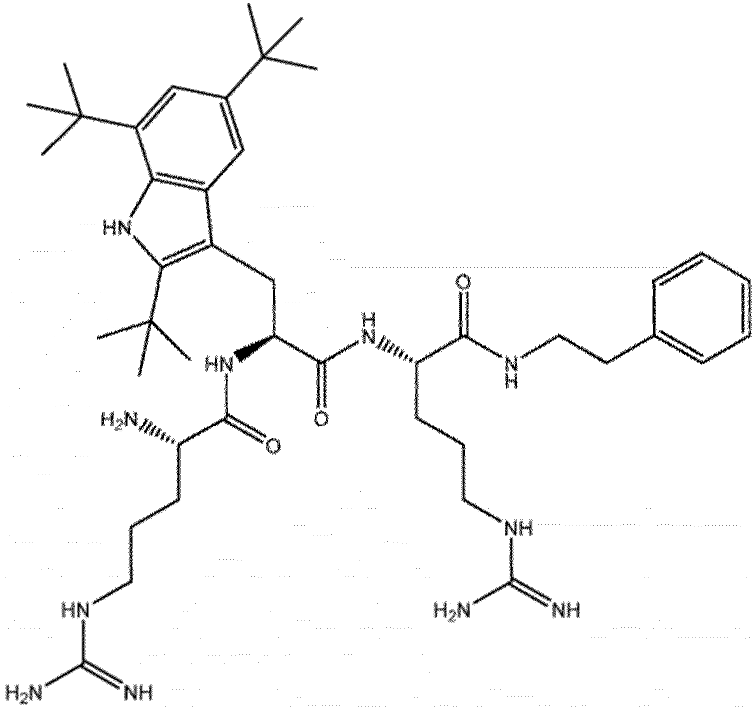

14. Verfahren nach einem der vorstehenden Ansprüche, wobei die Aminosäure oder das Peptid des zweiten Molekülteils in Form eines Salzes bereitgestellt wird.

15. Verbindung der Formel (I), wie in einem der Ansprüche 1 oder 3-5 definiert.

## Revendications

1. Procédé de fabrication d'un peptide cible comprenant la mise en réaction d'un composé anhydride mixte de Formule (I) et d'un second fragment qui est un acide aminé ou un peptide ;
dans lequel la Formule (I) présente la structure : 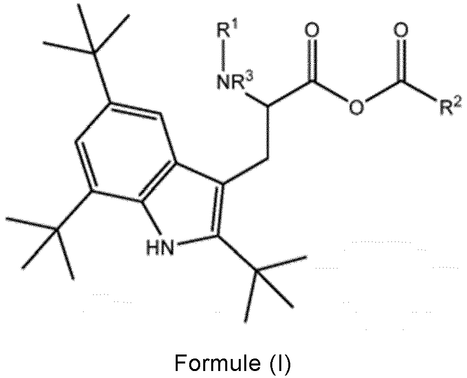
dans lequel R¹ est un groupe protecteur, un peptide ou un acide aminé ;
dans lequel R² est *tert*-butyle, isobutoxy, *tert*-butoxy, isobutyle, isopropoxy, isopropyle ou éthoxy ; et
dans lequel R³ est H ou un groupe alkylsilyle.

2. Procédé selon la revendication 1 comprenant la préparation du composé anhydride mixte de Formule (I) par mise en réaction d'un premier fragment de Formule (II) et d'un activateur de Formule (III) en présence d'une base ;
dans lequel la Formule (II) présente la structure : 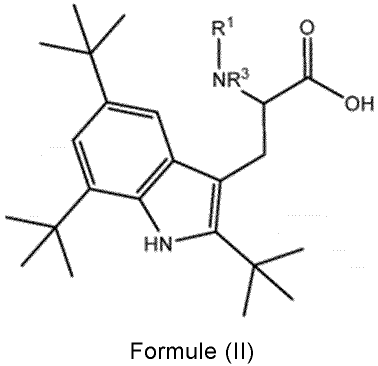 dans lequel R¹ est un groupe protecteur, un peptide ou un acide aminé et R³ est H ou un groupe alkylsilyle ;
et dans lequel la Formule (III) présente la structure : 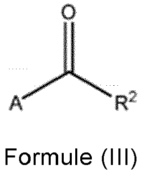
dans lequel R² est *tert-butyle,* isobutoxy, *tert-butoxy,* isobutyle, isopropoxy, isopropyle ou éthoxy ; et
A est un halogène.

3. Procédé selon la revendication 1 ou la revendication 2, dans lequel
(A) R³ est H ; et/ou
(B) R¹ est un groupe protecteur choisi dans benzyloxycarbonyle (Cbz), *tert-*butoxycarbonyle
(Boc), 4-méthoxy-2,3,6-triméthylbenzène sulfonyle (Mtr), 9-fluorenyl méthoxy carbonyle
(Fmoc) et 2,2,2-trichloroéthoxycarbonyle (Troc) ; un peptide ou un acide aminé ; et/ou
(C) R¹ est un peptide ou un acide aminé, de préférence dans lequel R¹ est un acide aminé.

4. Procédé selon l'une quelconque des revendications précédentes, dans lequel :
(A) R² est *tert*-butyle ou isobutoxy, de préférence *tert-butyle* ; ou
(B) R² est *tert*-butyle, isobutyle ou isopropyle.

5. Procédé selon l'une quelconque des revendications précédentes dans lequel l'acide aminé R¹ est un acide aminé cationique AA₁, de préférence dans lequel AA₁ est arginine.

6. Procédé selon l'une quelconque des revendications 2 à 5, comprenant en outre la préparation d'un composé de Formule (II), de préférence dans lequel R¹ est un peptide ou un acide aminé et le procédé comprend le couplage dudit peptide ou acide aminé à un résidu de tri-tert-butyl-tryptophane (Tbt).

7. Procédé selon l'une quelconque des revendications précédentes dans lequel le second fragment comprend un ou plusieurs groupes protecteurs et/ou un groupe de coiffage terminal en C.

8. Procédé selon la revendication 7, dans lequel le groupe de coiffage terminal en C est de formule -X-Y-Z, dans lequel :
X est un atome N, qui peut être substitué par un groupe alkyle ou aryle en C₁-C₁₀ ramifié ou non ramifié, et ce groupe alkyle ou aryle peut incorporer jusqu'à 2 hétéroatomes choisis dans N, O et S ;
Y représente un groupe choisi dans -Rₐ-R_{b}-, -Rₐ₋R_{b}-R_{b}- et -R_{b}-R_{b}-Rₐ₋ dans lequel
Rₐ est C, O, S ou N, et
R_{b} est C ; chacun de Rₐ et R_{b} peut être substitué par des groupes alkyle en C₁-C₄ ou non substitué ; et
Z est un groupe comprenant entre 1 et 3 groupes cycliques chacun de 5 ou 6 atomes autres qu'hydrogène, 2 ou plusieurs des groupes cycliques peuvent être fusionnés et un ou plusieurs des groupes cycliques peuvent être substitués ; le fragment Z incorpore un maximum de 15 atomes autres qu'hydrogène ; et dans lequel
la liaison entre Y et Z est une liaison covalente entre Rₐ ou R_{b} de Y et un atome autre qu'hydrogène de l'un des groupes cycliques de Z.

9. Procédé selon l'une quelconque des revendications précédentes, dans lequel le second fragment est un acide aminé comprenant AA₃, ou un peptide comprenant AA₃ comme l'acide aminé terminal en N, dans lequel AA₃ est un acide aminé cationique.

10. Procédé selon l'une quelconque des revendications précédentes, dans lequel le second fragment est un composé de Formule (IV)
AA₃-X-Y-Z (IV)
dans lequel :
AA₃ est un acide aminé cationique ;
X est un atome N, qui peut être substitué par un groupe alkyle ou aryle en C₁-C₁₀ ramifié ou non ramifié, et ce groupe alkyle ou aryle peut incorporer jusqu'à 2 hétéroatomes choisis dans N, O et S ;
Y représente un groupe choisi dans -Rₐ-R_{b}-, -Rₐ₋R_{b}-R_{b}- et -R_{b}-R_{b}-Rₐ₋ dans lequel
Rₐ est C, O, S ou N, et
R_{b} est C ; chacun de Rₐ et R_{b} peut être substitué par des groupes alkyle en C₁-C₄ ou non substitué ; et
Z est un groupe comprenant entre 1 et 3 groupes cycliques chacun de 5 ou 6 atomes autres qu'hydrogène, 2 ou plusieurs des groupes cycliques peuvent être fusionnés et un ou plusieurs des groupes cycliques peuvent être substitués ; le fragment Z incorpore un maximum de 15 atomes autres qu'hydrogène ; et dans lequel
la liaison entre Y et Z est une liaison covalente entre Rₐ ou R_{b} de Y et un atome autre qu'hydrogène de l'un des groupes cycliques de Z.

11. Procédé selon la revendication 9 ou la revendication 10, dans lequel AA₃ est lysine et/ou arginine, de préférence arginine.

12. Procédé selon l'une quelconque des revendications 8 à 11 dans lequel :
(A) X est non substitué ; et/ou
(B) Rₐ est C ; et/ou
(C) Y est -Rₐ₋R_{b}- et non substitué, de préférence dans lequel Y est -CH₂-CH₂- ; et/ou
(D) Z est phényl.

13. Procédé selon l'une quelconque des revendications précédentes, dans lequel le peptide cible présente la structure :

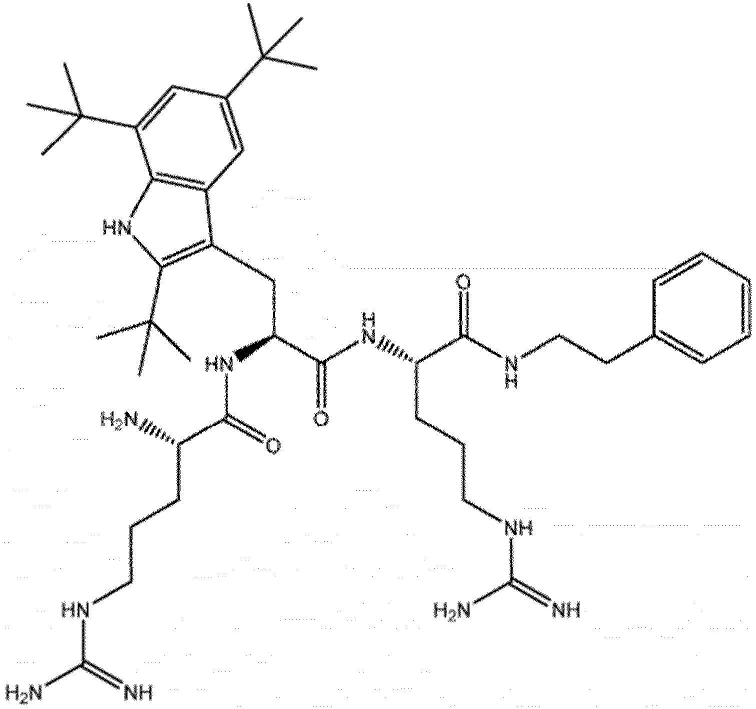

14. Procédé selon l'une quelconque des revendications précédentes, dans lequel l'acide aminé ou le peptide du second fragment est fourni sous la forme d'un sel.

15. Composé de Formule (I) tel que défini dans l'une quelconque des revendications 1 ou 3 à 5.
